(19) Europäisches Patentamt
European Patent Office
Office européen des brevets

(11) Veröffentlichungsnummer: **0 097 122**
**B1**

(12) # EUROPÄISCHE PATENTSCHRIFT

(45) Veröffentlichungstag der Patentschrift: **31.05.89**

(51) Int. Cl.⁴: **C 07 D 401/12**, C 07 D 409/12, C 07 D 403/12, C 07 D 407/12, A 01 N 47/36

(21) Anmeldenummer: **83810248.1**

(22) Anmeldetag: **08.06.83**

(54) **N-Heterocyclosulfonyl-N'-pyrimidinyl- und -triazinylharnstoffe.**

(30) Priorität: **14.06.82 CH 3671/82**

(43) Veröffentlichungstag der Anmeldung:
**28.12.83 Patentblatt 83/52**

(45) Bekanntmachung des Hinweises auf die Patenterteilung:
**31.05.89 Patentblatt 89/22**

(84) Benannte Vertragsstaaten:
**AT BE CH DE FR GB IT LI NL SE**

(56) Entgegenhaltungen:
**EP-A-0 009 419**
**EP-A-0 013 480**
**EP-A-0 030 142**
**EP-A-0 039 239**
**EP-A-0 070 804**

(73) Patentinhaber: **CIBA- GEIGY AG, Klybeckstrasse 141, CH- 4002 Basel (CH)**

(72) Erfinder: **Böhner, Beat, Dr., Hügelweg 3, CH- 4102 Binningen (CH)**
Erfinder: **Föry, Werner, Dr., Benkenstrasse 65, CH- 4054 Basel (CH)**
Erfinder: **Gass, Karl, Blumenrain 4, CH- 4312 Magden (CH)**
Erfinder: **Meyer, Willy, Talweg 49, CH- 4125 Riehen (CH)**

**EP 0 097 122 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die vorliegende Erfindung betrifft neue, herbizid wirksame und pflanzenwuchsregulierende N-Heterocyclosulfonyl-N'-pyrimidinyl- und -triazinylharnstoffe, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmen des Pflanzenwachstums.

Die erfindungsgemässen N-Heterocyclosulfonyl-N'-pyrimidinyl- und -triazinylharnstoffe entsprechen der allgemeinen Formel

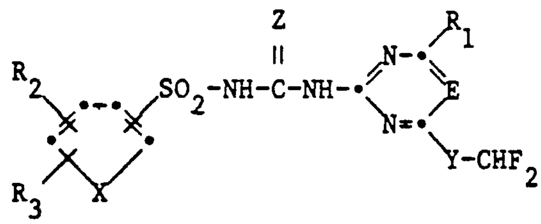

(I)

worin

X Sauerstoff, Schwefel, $-N(R_4)-$ oder $-CN=$ mit $R_5$ (d. h. $-C(R_5)=N-$),

Y Sauerstoff oder Schwefel,

Z Sauerstoff oder Schwefel,

E Stickstoff oder $-CH=$,

$R_1$ Wasserstoff, $C_1$-$C_4$-Alkyl, $C_1$-$C_4$-Halogenalkyl, $C_1$-$C_4$-Halogenalkoxy, $C_1$-$C_4$-Alkoxy, Halogen, $-NR_6R_7$ oder Alkoxyalkyl mit höchstens 4 Kohlenstoffatomen,

$R_2$ Wasserstoff, $C_1$-$C_3$-Alkyl, $C_1$-$C_3$-Halogenalkyl, Halogen, Nitro, $C_1$-$C_3$-Alkoxy, $-C(=W)-R_8$, $-SO_2-NR_6R_7$, $-SO_n-C_1-C_3$-Alkyl oder $-CO-R_9$

$R_3$ Wasserstoff, Halogen, $C_1$-$C_3$-Alkyl, Methoxy, Nitro oder Trifluormethyl,

$R_4$ Wasserstoff, $C_1$-$C_4$-Alkyl, Cyanoalkyl mit höchstens 4 Kohlenstoffatomen, $C_3$-$C_6$-Cycloalkyl, Benzyl, $-CO-C_1-C_4$-Alkoxy, $-CO-NR_6R_7$ oder gegebenenfalls durch 1 bis 3 Halogenatome substituiertes $-CO-C_1-C_4$-Alkyl,

$R_5$ Wasserstoff, Nitro, Fluor, Chlor, Brom, Methyl, Trifluormethyl, $-SO_n-C_1-C_3$-Alkyl, $-CO-C_1-C_4$-Alkoxy oder $C_1$-$C_3$-Alkoxy,

$R_6$ Wasserstoff, $C_1$-$C_6$-Alkyl, Cyanoalkyl mit höchstens 4 Kohlenstoffatomen, Methoxy oder Äthoxy,

$R_7$ Wasserstoff, $C_1$-$C_6$-Alkyl oder $C_3$-$C_6$-Alkenyl oder

$R_6$ und $R_7$ zusammen mit dem sie bindenden Stickstoffatom einen 5- bis 6-gliedrigen gesättigten, gegebenenfalls ein Sauerstoff- oder Schwefelatom als Ringglied enthaltenden, Heterocyclus,

$R_8$ Wasserstoff, $C_1$-$C_6$-Alkyl, $C_1$-$C_4$-Halogenalkyl, $C_3$-$C_6$-Cycloalkyl, $C_4$-$C_7$-Cycloalkylalkyl oder Alkoxyalkyl mit höchstens 4 Kohlenstoffatomen,

$R_9$ $C_1$-$C_6$-Alkoxy, $C_3$-$C_6$-Alkenyloxy, $C_3$-$C_6$-Alkinyloxy, $C_2$-$C_6$-Halogenalkoxy, $C_1$-$C_4$-Cyanoalkoxy, $C_1$-$C_6$-Alkylthio, $C_3$-$C_6$-Alkenylthio, $C_3$-$C_6$-Alkinylthio, $C_5$-$C_6$-Cycloalkoxy, $C_4$-$C_7$-Cycloalkylalkoxy, $-NR_6R_7$- oder Alkoxyalkoxy mit höchstens 6 Kohlenstoffatomen,

W Sauerstoff oder $=N-O-R_{10}$, worin $R_{10}$ für Wasserstoff, $C_1$-$C_6$-Alkyl oder $C_3$-$C_6$-Alkenyl steht, und

n eine Zahl von Null bis zwei bedeuten, sowie die Salze dieser Verbindungen.

Harnstoffverbindungen, Triazinverbindungen und Pyrimidinverbindungen mit herbizider Wirkung sind allgemein bekannt. Kürzlich wurden Arylsulfonyl-heterocyclyl-harnstoffverbindungen mit herbizider und pflanzenwuchsregulierender Wirkung, beispielsweise in den europäischen Patentpublikationen Nr. 9 419, 13 480, 30 142 oder 39 239 oder in der DE-OS-2 715 786 beschrieben.

In den Definitionen ist unter Alkyl geradkettiges oder verzweigtes Alkyl zu verstehen; z. B. Methyl, Äthyl, n-Propyl, i-Propyl, die isomeren Butyl-, Pentyl- oder Hexylreste.

Unter Alkoxy ist im allgemeinen zu verstehen: Methoxy, Äthoxy, n-Propyloxy i-Propyloxy, die vier isomeren Butyloxyreste, n-Amyloxy, i-Amyloxy, 2-Amyloxy oder 3-Amyloxy, insbesondere aber Methoxy, Äthoxy oder i-Propyloxy.

Beispiele für Alkylthio sind Methylthio, Äthylthio, n-Propylthio, i-Propylthio, n-Butylthio oder n-Pentylthio insbesondere aber Methylthio und Äthylthio.

Unter Cycloalkyl sind im Rahmen der vorliegenden Anmeldung Cyclopropyl, Cyclobutyl, Cyclopentyl und Cyclohexyl zu verstehen.

Beispiele für Cycloalkylalkyl sind Cyclopropylmethyl, Cyclopropyläthyl, Cyclopropylpropyl, Cyclobutylmethyl, Cyclobutyläthyl, Cyclopentylmethyl, Cyclopentyläthyl und Cyclohexylmethyl. Bevorzugt sind Cyclopentylmethyl, Cyclohexylmethyl und Cyclopropylmethyl.

Bevorzugte Cyanoalkylreste sind im allgemeinen Cyanomethyl, Cyanoäthyl und Cyanopropyl.

Heterocyclen, die von den Resten $R_6$ und $R_7$ zusammen mit dem Stickstoffatom gebildet werden können sind vorzugsweise Pyrrolidin, Piperidin, Morpholin oder Thiomorpholin.

Als Alkoxyalkylreste innerhalb oder obigen Definitionen sind bevorzugt: Methoxymethyl, Methoxyäthyl, Äthoxymethyl und Äthoxyäthyl.

Durch Definition des Symbols X werden als Heterocyclen, die über die Sulfongruppe gebunden sind umfasst: Furan, Thiophen, Pyrrol und Pyridin.

Beispiele für Alkenylreste sind Vinyl, Allyl, Isopropenyl, 1-Propenyl, 1-Butenyl, 2-Butenyl, 3-Butenyl, 1-Isobutenyl, 2-Isobutenyl, 1-Pentenyl, 2-Pentenyl, 3-Pentenyl, 4-Pentenyl sowie die isomeren Hexenylreste, insbesondere aber Vinyl, Allyl und 4-Pentenyl.

Beispiele für Alkylsulfinyl sind Methylsulfinyl, Äthylsulfinyl, n-Propylsulfinyl und n-Butylsulfinyl, insbesondere aber Methylsulfinyl und Äthylsulfinyl.

Beispiele für Alkylsulfonyl sind Methylsulfonyl, Äthylsulfonyl oder n-Propylsulfonyl, insbesondere aber Methylsulfonyl und Äthylsulfonyl.

Unter Halogen in den Definitionen sowie als Teil in Halogenalkoxy, Halogenalkyl oder Halogenalkylthio sind Fluor, Chlor und Brom, vorzugsweise jedoch Fluor und Chlor zu verstehen.

Alkinylresten in den Definitionen der obigen Symbole entsprechen in der Regel Propargyl, 2-Butinyl, 3-Butinyl sowie isomere Pentinylreste, vorzugsweise ist der Alkinylrest jedoch durch Propargyl oder 2- oder 3-Butinyl verkörpert.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeigneter Amine sind primäre, sekundäre und tertiäre aliphatische und aromatische Amine wie Methylamin, Äthylamin, Propylamin, i-Propylamin, die vier isomeren Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Äthyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z. B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Von den erfindungsgemässen Verbindungen der Formel I sind die Verbindungen bevorzugt, in denen entweder

a) Z Sauerstoff oder
b) Y Sauerstoff oder
c) X Schwefel, $-NR_4-$ oder $-CR_5=N-$ oder
d) E die Methinbrücke -CH= oder
e) $R_1$ Chlor, Dimethylamino, Trifluormethyl, Fluormethyl, Methoxy, Difluormethoxy, Methyl oder Äthyl oder
f) $R_3$ Wasserstoff bedeuten oder
g) der Rest $R_2$ in Nachbarstellung zur Sulfonylgruppe steht.

Unter den Verbindungen der Untergruppe g) geniessen die jenigen eine weitere Bevorzugung, in denen $R_2$ für Wasserstoff, Fluor, Chlor, Methoxy, Acetyl, $-COOCH_2-CH=CH_2$, $COOCH_2-C=CH$, $-SO_2-N(CH_3)_2$, $-SO_2-CH_3$ oder $-CO-C_1-C_4$-Alkoxy steht.

Eine weitere bevorzugte Untergruppe bilden die Verbindungen der Formel I, in denen Y und Z Sauerstoff, E die Methinbrücke, $R_1$ Chlor, Dimethylamino,Trifluormethyl, Fluormethyl, Methoxy, Difluormethoxy, Methyl oder Äthyl bedeuten, $R_2$ sich in Nachbarstellung zur Sulfonylgruppe befindet und für Wasserstoff, Fluor, Chlor, Methoxy, Acetyl, $-SO_2-CH_3$, $-COOCH_2-CH=CH_2$, $-COOCH_2-C\equiv CH$, $-SO_2-N(CH_3)_2$ oder $-CO-C_1-C_4$-Alkoxy steht und $R_3$ Wasserstoff bedeutet.

Ganz besonders bevorzugte Untergruppen von Wirkstoffen der Formel I bilden diejenigen Verbindungen, in denen X für ein Schwefelatom steht, Y und Z Sauerstoff, E die Methinbrücke, R Chlor, Dimethylamino, Trifluormethyl, Fluormethyl, Methoxy, Difluormethoxy, Methyl oder Äthyl bedeuten, $R_2$ sich in Nachbarstellung zur Sulfonylgruppe befindet und für Wasserstoff, Fluor, Chlor, Methoxy, Acetyl, $-SO_2-CH_3$, $-COOCH_2-CH=CH_2$, $-COOCH_2-C\equiv CH$, $-SO_2-N(CH_3)_2$ oder $-CO-C_1-C_4$-Alkoxy steht und R Wasserstoff bedeutet; diejenigen, in denen X für Schwefel oder -CH=N- steht, Y und Z Sauerstoff, E die Methinbrücke, $R_1$ Chlor, Dimethylamino, Fluormethyl, Methoxy, Difluormethoxy, Methyl oder Äthyl bedeuten, $R_2$ sich in Nachbarstellung zur Sulfonylgruppe befindet und für Wasserstoff, Chlor, Acetyl, $-SO_2-CH_3$, $-COOCH_2-CH=CH_2$, $-COOCH_2-C\equiv CH$ oder $-CO-C_1-C_4$-Alkoxy steht und $R_3$ Wasserstoff bedeutet und diejenigen, in denen X für $-CR_5=N-$ steht, Y und Z Sauerstoff, E die Methinbrücke, $R_1$ Chlor, Dimethylamino, Trifluormethyl, Fluormethyl, Methoxy,

Difluormethoxy, Methyl oder Äthyl bedeuten, $R_2$ sich in Nachbarstellung zur Sulfonylgruppe befindet und für Wasserstoff, Fluor, Chlor, Methoxy, Acetyl, $-SO_2-CH_3$, $-COOCH_2-CH=CH_2$, $-COOCH_2-C\equiv CH$, $-SO_2-N(CH_3)_2$ oder -CO-$C_1$-$C_4$-Alkoxy steht und $R_3$ Wasserstoff bedeutet.

Als bevorzugte Einzelverbindungen sind zu nennen:

N-(2-Methoxycarbonyl-3-thienylsulfonyl)-N'-(4-difluormethoxy-6-methylpyrimidin-2-yl)-harnstoff,
N-(4-Methoxycarbonyl-3-thienylsulfonyl)-N'-(4-difluormethoxy-6-methoxypyrimidin-2-yl)-harnstoff,
N-(4-Methoxycarbonyl-3-thienylsulfonyl)-N'-(4-difluormethoxy-6-methylpyrimidin-2-yl)-harnstoff,
N-(2-Chlor-3-pyridylsulfonyl)-N'-(4-difluormethoxy-6-methylpyrimidin-2-yl)-harnstoff,
N-(2-Pyridinylsulfonyl)-N'-(4-difluormethoxy-6-methylpyrimidin-2-yl)-harnstoff,
N-(2-Pyrrolylsulfonyl)-N'-(4-difluormethoxy-6-methylpyrimidin-2-yl)-harnstoff und
N-(3-Pyrrolylsulfonyl)-N'-(4-difluormethoxy-6-methylpyrimidin-2-yl)-harnstoff.

Die Herstellung der Verbindungen der Formel I erfolgt in einem inerten, organischen Lösungsmittel.

Nach einem ersten Verfahren werden die Verbindungen der Formel I erhalten, indem man ein Heterocyclosulfonamid der Formel II

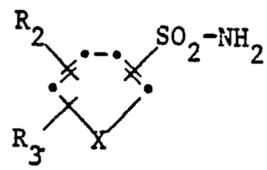 (II),

worin X, $R_2$ und $R_3$ die unter Formel I gegebene Bedeutung haben, in Gegenwart einer Base mit einem N-Pyrimidinyl- oder -triazinylcarbamat der Formel III

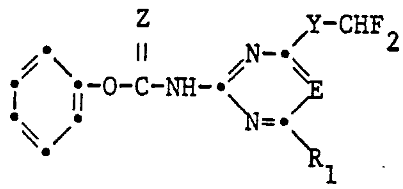 (III),

worin E, $R_1$, Y und Z die unter Formel I gegebene Bedeutung haben, umsetzt.

Nach einem zweiten Verfahren gelangt man zu Verbindungen der Formel I, indem man ein Heterocyclosulfonylisocyanat oder -isothiocyanat der Formel IV

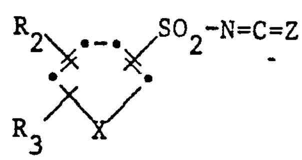 (IV),

worin $R_2$, $R_3$, X und Z die unter Formel I gegebene Bedeutung haben, gegebenenfalls in Gegenwart einer Base, mit einem Aminopyrimidin oder -triazin der Formel V

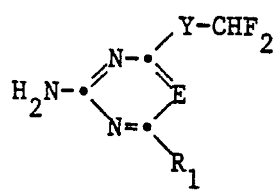 (V),

worin E, $R_1$ und Y die unter Formel I gegebene Bedeutung haben, umsetzt.

Nach einem weiteren Verfahren werden die Verbindungen der Formel I hergestellt, indem man ein Heterocyclosulfonamid der oben angegebenen Formel II gegebenenfalls in Gegenwart einer Base mit einem Isocyanat oder Isothiocyanat der Formel VI

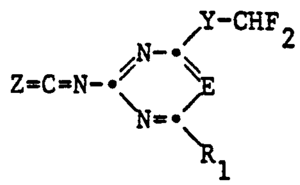

(VI),

worin E, $R_1$, Y und Z die unter Formel I gegebene Bedeutung haben, umsetzt.

Schliesslich kann man die Verbindungen der Formel I auch erhalten, indem man ein N-Heterocyclosulfonylcarbamat der Formel VII

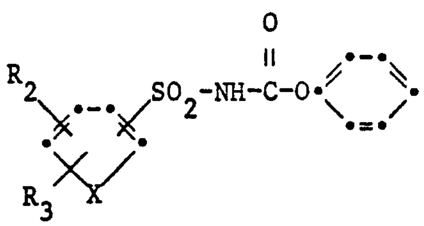

(VII),

worin $R_2$, $R_3$ und X die unter Formel I gegebene Bedeutung haben, mit einem Aminopyrimidin oder -triazin der oben angegebenen Formel V umsetzt.

Die erhaltenen Harnstoffe der Formel I können gewünschtenfalls mittels Aminen, Alkalimetall- oder Erdalkalimetallhydroxiden oder quaternären Ammoniumbasen in Additionssalze übergeführt werden. Dieses geschieht beispielsweise durch Umsetzen mit der äquimolaren Menge Base und Verdampfen des Lösungsmittels.

Diese Umsetzungen zu Verbindungen der Formel I werden vorteilhafterweisein aprotischen, inerten, organischen Lösungsmitteln vorgenommen wie Methylenchlorid, Tetrahydrofuran, Acetonitril, Dioxan, Toluol.

Die Reaktionstemperaturen liegen vorzugsweise zwischen -20° und +120° C. Die Umsetzumgen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator verkürzt werden.

Als Basen können sowohl organische Basen wie Amine, wie Triäthylamin, Chinuclidin, Pyridin etc. als auch anorganische Basen wie Hydride wie Natrium- oder Calciumhydrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden.

Die Endprodukte können durch Einengen und/oder Verdampfen des Lösungsmittels isoliert und durch Umkristallisieren oder Zerreiben des festen Rückstandes in Lösungsmitteln in denen sie sich nicht gut lösen, die Äther, aromatischen Kohlenwasserstoffen oder chlorierten Kohlenwasserstoffen gereinigt werden.

Die Wirkstoffe der Formel I sind stabile Verbindungen. Ihre Handhabung bedarf keine vorsorglicher Massnahmen.

Die Zwischenprodukte der Formeln II, IV und VII sind bekannt oder können analog zu bekannten Verfahren hergestellt werden.

Verbindungen der Formel V werden gemäss der europäischen Patentanmeldung Nr. 70 804 erhalten, indem man ein Aminopyrimidin oder -triazin der Formel VIII

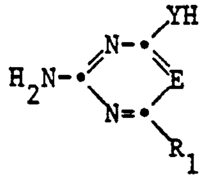

(VIII)

worin $R_1$, Y und E die unter Formel I gegebene Bedeutung haben in Gegenwart einer Base mit Difluorchlormethan oder Difluor-brommethan umsetzt.

Das Verfahren zur Herstellung der Verbindungen der Formel V wird mit Vorteil in einem inerten polaren Lösungsmittel oder Lösungsmittelgemisch ausgeführt. Geeignete Lösungsmittel sind Äther wie Dioxan, Tetrahydrofuran, Äthylenglykoldimethyläther, Diäthylenglykoldimethyläther; Alkohole wie Methanol, Äthanol; Ketone wie Aceton, Äthylmethylketon; Dimethylformamid; Acetonitril oder Dimethylsulfoxid. Als Basen sind in

besonderer Weise geeignet; Natrium- und Calciumhydrid, Kalium- und Natriumhydroxid, Kalium- und Natriumcarbonat. In geeigneten Fällen kann die Base als wässrige Lösung zugesetzt werden.

Die Ausgangsmaterialien der Formel VIII sind bekannt oder werden analog zu bekannten Verfahren erhalten.

Die ebenfalls Zwischenprodukte der Formeln III und VI werden analog zu bekannten Verfahren aus den Zwischenprodukten der Formel V hergestellt.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatorensehr geringen Aufwandmengen.

Die Verbindungen der Formel I haben ausserdem starke pflanzenwuchsregulierende, insbesondere pflanzenwuchshemmende, Eigenschaften. Es werden sowohl Monokotyledonen als auch Dikotyledonen in ihrem Wachstum beeinträchtigt.

So können z. B. die in der Landwirtschaft in tropischen Gegenden häufig als "cover crops" (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Weiter eignen sich die Verbindungen der Formel I um das Keimen von eingelagerten Kartoffeln zu verhindern. Bei Kartoffeln entwickeln sich bei der Einlagerung über den Winter häufig Keime, die Schrumpfen, Gewichtsverlust und Faulen zur Folge haben.

Bei grösseren Aufwandmengen werden alle getesteten Pflanzem in ihrer Entwicklung so geschädigt, dass sie absterben.

Die Erfindung betrifft auch herbizide unf pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsiönen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorganulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z. B. die Na- oder K-Salze der Öl- oder Stearinsäure, oder von natürlichen Fettsäuregemischen, die z. B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2-Sulfonsäuregruppen und einen Fettsäurerest mit 8 - 22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Äthylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigen Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl oder niedrige Hydroxyalkylreste aufweisen. Die Salze liegen vorzugsweise als Halogenide, Methylsulfate oder Äthylsufate vor, z. B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979. Sisley and Wood, "Encyclopedia of Surface Active Agents". Chemical Publishing Co., Inc. New York, 1964.

Die pestiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

<u>Emulgierbare Konzentrate</u>
Aktiver Wirkstoff: 1 bis 20 %, bevorzugt 5 bis 10 %
oberflächenaktives Mittel: 5 bis 30 %, vorzugsweise 10 bis 20 %
flüssiges Trägermittel: 50 bis 94 %, vorzugsweise 70 bis 85 %.

<u>Stäube</u>
Aktiver Wirkstoff: 0,1 bis 10 %, vorzugsweise 0,1 bis 1 %
festes Trägermittel: 99,9 bis 90 %, vorzugsweise 99,9 bis 99 %.

<u>Suspension-Konzentrate</u>
Aktiver Wirkstoff: 5 bis 75 %, vorzugsweise 10 bis 50 %
Wasser: 94 bis 25 %, vorzugsweise 90 bis 30 %
oberflächenaktives Mittel: 1 bis 40 %, vorzugsweise 2 bis 30 %.

<u>Benetzbare Pulver</u>
Aktiver Wirkstoff: 0,5 bis 90 %, vorzugsweise 1 bis 80 %
oberflächenaktives Mittel: 0,5 bis 20 %, vorzugsweise 1 bis 15 %
festes Trägermittel: 5 bis 95 %, vorzugsweise 15 bis 90 %.

<u>Granulate</u>
Aktiver Wirkstoff: 0,5 bis 30 %, vorzugsweise 3 bis 15 %

festes Trägermittel: 99,5 bis 70 %, vorzugsweise 97 bis 85 %.

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,025 bis 5 kg AS/ha.

**Herstellungsbeispiele:**

**Beispiel 1:**

N-(2-Methoxycarbonyl-3-thienylsulfonyl)-N'-(4-difluormethoxy-6-methylpyrimidin-2-yl)-harnstoff

Zur Lösung von 4,4 g 2-Amino-4-difluormethoxy-6-methylpyrimidin in 80 ml absolutem Tetrahydrofuran lässt man unter Rühren eine Lösung von 6,2 g 2-Methoxycarbonyl-3-thienylsulfonylisocyanat in 50 ml absolutem Tetrahydrofuran zutropfen. Die Temperatur des Reaktionsgemisches erhöht sich dabei von 20°C auf 26°C. Nach weiterem Rühren für eine Stunde, wird das Reaktionsgemisch filtriert und eingedampft. Durch Kristallisation des Rückstandes aus Äthylacetat erhält man 5,3 g (50 %) N-(2-Methoxycarbonyl-3-thienylsulfonyl)-N'-(4-difluormethoxy-6-methylpyrimidin-2-yl)-harnstoff. Smp. 178 - 179°C (Verbindung Nr. 2.1).

**Beispiel 2:**

N-(2-Chlor-3-pyridinylsulfonyl)-N'-(4-difluormethoxy-6-methylpyrimidin-2-yl)-harnstoff

Einem Gemisch von 0,652 g 2-Chlor-3-pyridinylsulfonamid, 0,55 ml 1,8-Diazabicyclo[5.4.0]undec-7-en in 10 ml absolutem Dioxan setzt man bei 25°C 1,0 g N-(4-Difluormethoxy-6-methylpyrimidin-2-yl)-phenylcarbamat zu. Die Reaktionsmischung wird für weitere 1,5 Stunden bei der gleichen Temperatur gerührt und anschliessend eingedampft. Der Rückstand wird mit Eiswasser versetzt und mit 1,86 ml 2N Salzsäure neutralisiert. Der entstehende Niederschlag wird abgetrennt, mit Wasser gewaschen und getrocknet. Man erhält so 1,2 g (90 %) N-(2-Chlor-3-pyridinylsulfonyl)-N'-(4-difluormethoxy-6-methylpyrimidin-2-yl)-harnstoff, Smp. 161 - 162°C (Verbindung Nr. 1.15).

**Beispiel 3:**

N-(2-Pyridinylsulfonyl)-N'-(4-difluormethoxy-6-methylpyrimidin-2-yl)-harnstoff

Eine Lösung von 0,52 g 2-Pyridinylsulfonamid und 0,55 g 1,8-Diazabicyclo[5.4.0]undec-7-en in 10 ml absolutem Dioxan wird mit 1,0 g N-(4-Difluormethoxy-6-methylpyrimidin-2-yl)-phenylcarbamat versetzt und für 6 Stunden bei 20 - 25°C gerührt. Anschliessend wird das Reaktionsgemisch mit Wasser aufgenommen und mit 2N Salzsäure angesäuert. Der entstehende Niederschlag wird abgetrennt, mit Wasser gewaschen und aus einem Aceton/Äther-Gemisch umkristallisiert. Man erhält so 0,5 g (42,4 %) N-(2-Pyridinylsulfonyl)-N'-(4-difluormethoxy-6-methylpyrimidin-2-yl)-harnstoff, Smp. 150 - 151°C (Verbindung Nr. 1.1)

**Beispiel 4:**

N-Pyrrolylsulfonyl-N'-(4-difluormethoxy-6-methylpyrimidin-2-yl)-harnstoff

Eine Lösung von 7,0 g 2-Amino-4-difluormethoxy-6-methylpyrimidin in 100 ml absolutem Tetrahydrofuran wird bei 0 - 5°C tropfenweise mit 5,7 g Chlorsulfonylisocyanat versetzt. Nachdem entstandene gelbliche Lösung für 10 Minuten bei 0°C gerührt worden ist, werden 5,6 ml Pyrrol zugesetzt und das Reaktionsgemisch wird für 17 Stunden bei 20 - 25°C gerührt. Nach Abdampfen des Lösungsmittels wird der Rückstand mit einem Gemisch von 25 ml Methanol, 50 ml Wasser und 3,2 g Natriumhydroxid aufgenommen, für 10 Minuten kräftig geschüttelt, zweimal mit je 50 ml Methylenchlorid gewaschen und mit Eisessig angesäuert. Danach wird die saure wässrige Lösung mit Äthylacetat extrahiert. Die organischen Extrakte werden mit Magnesiumsulfat getrocknet und eingedampft. Dabei erhält man in 20-%-iger Ausbeute ein 1 : 1-Gemisch von zwei isomeren Produkten. Dieses Isomerengemisch wird mit Petroläther/Äthylacetat-Gemisch (4 : 1) an Kieselgel chromatographiert. Man erhält so N-(2-Pyrrolylsulfonyl)-N'-(4-difluormethoxy-6-methylpyrimidin-2-yl)-harnstoff, Smp. 189°C (Verbindung Nr. 4.1) und N-(3-Pyrrolylsulfonyl)-N'-(4-difluormethoxy-6-methylpyrimidin-2-yl)-harnstoff, Smp. 173 - 174°C (Verbindung Nr. 4.8).

In analoger Weise werden die in den folgenden Tabellen 1 bis 4 aufgelisteten Verbindungen der Formel I erhalten.

**Tabelle 1:**

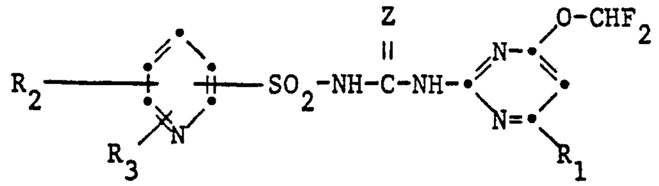

| Verb. Nr. | $R_1$ | $R_2$ | Stellung der Sulfonylgruppe | $R_3$ | Z | Smp. [°C] |
|---|---|---|---|---|---|---|
| 1.1 | $CH_3$ | H | 2 | H | O | 150 - 151 |
| 1.2 | $OCH_3$ | H | 2 | H | O | |
| 1.3 | Cl | H | 2 | H | O | |
| 1.4 | $-N(CH_3)_2$ | H | 2 | H | O | |
| 1.5 | $C_2H_5$ | H | 2 | H | O | |
| 1.6 | $CF_3$ | H | 2 | H | O | |
| 1.7 | $-OCHF_2$ | H | 2 | H | O | |
| 1.8 | $CH_3$ | H | 3 | H | O | |
| 1.9 | $OCH_3$ | H | 3 | H | O | |
| 1.10 | Cl | H | 3 | H | O | |
| 1.11 | $-N(CH_3)_2$ | H | 3 | H | O | |
| 1.12 | $C_2H_5$ | H | 3 | H | O | |
| 1.13 | $CF_3$ | H | 3 | H | O | |
| 1.14 | $-OCHF_2$ | H | 3 | H | O | |
| 1.15 | $CH_3$ | 2-Cl | 3 | H | O | 161 - 162 |
| 1.16 | $OCH_3$ | 2-Cl | 3 | H | O | |
| 1.17 | Cl | 2-Cl | 3 | H | O | |
| 1.18 | $-N(CH_3)_2$ | 2-Cl | 3 | H | O | |
| 1.19 | $C_2H_5$ | 2-Cl | 3 | H | O | |
| 1.20 | $CF_3$ | 2-Cl | 3 | H | O | |
| 1.21 | $-OCHF_2$ | 2-Cl | 3 | H | O | |
| 1.22 | $CH_3$ | $2-COOCH_3$ | 3 | H | O | |
| 1.23 | $OCH_3$ | $2-COOCH_3$ | 2 | H | O | |
| 1.24 | Cl | $2-COOCH_3$ | 3 | H | O | |
| 1.25 | $-N(CH_3)_2$ | $2-COOCH_3$ | 3 | H | O | |
| 1.26 | $C_2H_5$ | $2-COOCH_3$ | 3 | H | O | |
| 1.27 | $CF_3$ | $2-COOCH_3$ | 3 | H | O | |
| 1.28 | $-OCHF_2$ | $2-COOCH_3$ | 3 | H | O | |
| 1.29 | $CH_3$ | $2-SO_2-CH_3$ | 3 | H | O | 184 (Zers.) |
| 1.30 | $OCH_3$ | $2-SO_2-CH_3$ | 3 | H | O | |
| 1.31 | Cl | $2-SO_2-CH_3$ | 3 | H | O | |
| 1.32 | $-N(CH_3)_2$ | $2-SO_2-CH_3$ | 3 | H | O | |
| 1.33 | $C_2H_5$ | $2-SO_2-CH_3$ | 3 | H | O | |
| 1.34 | $CF_3$ | $2-SO_2-CH_3$ | 3 | H | O | |
| 1.35 | $OCHF_2$ | $2-SO_2-CH_3$ | 3 | H | O | |
| 1.36 | $CH_3$ | 2-F | 3 | H | O | |
| 1.37 | $OCH_3$ | 2-F | 3 | H | O | |
| 1.38 | Cl | 2-F | 3 | H | O | |
| 1.39 | $-N(CH_3)_2$ | 2-F | 3 | H | O | |
| 1.40 | $C_2H_5$ | 2-F | 3 | H | O | |
| 1.41 | $CF_3$ | 2-F | 3 | H | O | |
| 1.42 | $-OCHF_2$ | 2-F | 3 | H | O | |
| 1.43 | $CH_3$ | 3-Cl | 3 | H | O | |
| 1.44 | $OCH_3$ | 3-Cl | 2 | H | O | |
| 1.45 | Cl | 3-Cl | 2 | H | O | |
| 1.46 | $-N(CH_3)_2$ | 3-Cl | 2 | H | O | |
| 1.47 | $C_2H_5$ | 3-Cl | 2 | H | O | |

| | | | | | | |
|---|---|---|---|---|---|---|
| 1.48 | $CF_3$ | 3-Cl | 2 | H | O | |
| 1.49 | $-OCHF_3$ | 3-Cl | 2 | H | O | |
| 1.50 | $CH_3$ | $3-OCH_3$ | 2 | H | O | 161 (Zers.) |
| 1.51 | $OCH_3$ | $3-OCH_3$ | 2 | H | O | |
| 1.52 | Cl | $3-OCH_3$ | 2 | H | O | |
| 1.53 | $-N(CH_3)_2$ | $3-OCH_3$ | 2 | H | O | |
| 1.54 | $C_2H_5$ | $3-OCH_3$ | 2 | H | O | |
| 1.55 | $CF_3$ | $3-OCH_3$ | 2 | H | O | |
| 1.56 | $-OCHF_2$ | $3-OCH_3$ | 2 | H | O | |
| 1.57 | $CH_3$ | $2-COOCH_3$ | 3 | H | S | |
| 1.58 | $OCH_3$ | $2-COOCH_3$ | 3 | H | S | |
| 1.59 | Cl | $2-COOCH_3$ | 3 | H | S | |
| 1.60 | $-N(CH_3)_2$ | $2-COOCH_3$ | 3 | H | S | |
| 1.61 | $C_2H_5$ | $2-COOCH_3$ | 3 | H | S | |
| 1.62 | $CF_3$ | $2-COOCH_3$ | 3 | H | S | |
| 1.63 | $-OCHF_2$ | $2-COOCH_3$ | 3 | H | S | |
| 1.64 | $CH_3$ | $3-COOCH_3$ | 2 | H | S | |
| 1.65 | $OCH_3$ | $3-COOCH_3$ | 2 | H | S | |
| 1.66 | Cl | $3-COOCH_3$ | 2 | H | S | |
| 1.67 | $-N(CH_3)_2$ | $3-COOCH_3$ | 2 | H | S | |
| 1.68 | $C_2H_5$ | $3-COOCH_3$ | 2 | H | S | |
| 1.69 | $CF_3$ | $3-COOCH_3$ | 2 | H | S | |
| 1.70 | $-OCHF_2$ | $3-COOCH_3$ | 2 | H | S | |
| 1.71 | $CH_3$ | 6-F | 2 | H | O | |
| 1.72 | $OCH_3$ | 6-F | 2 | H | O | |
| 1.73 | Cl | 6-F | 2 | H | O | |
| 1.74 | $-N(CH_3)_2$ | 6-F | 2 | H | O | |
| 1.75 | $C_2H_5$ | 6-F | 2 | H | O | |
| 1.76 | $CF_3$ | 6-F | 2 | H | O | |
| 1.77 | $-OCHF_2$ | 6-F | 2 | H | O | |
| 1.78 | $CH_3$ | $6-OCH_3$ | 2 | H | O | |
| 1.79 | $OCH_3$ | $6-OCH_3$ | 2 | H | O | |
| 1.80 | Cl | $6-OCH_3$ | 2 | H | O | |
| 1.81 | $-N(CH_3)_2$ | $6-OCH_3$ | 2 | H | O | |
| 1.82 | $C_2H_5$ | $6-OCH_3$ | 2 | H | O | |
| 1.83 | $CF_3$ | $6-OCH_3$ | 2 | H | O | |
| 1.84 | $-OCHF_2$ | $6-OCH_3$ | 2 | H | O | |
| 1.85 | $CH_3$ | 4-Cl | 3 | H | O | 126 - 128 |
| 1.86 | $CH_3$ | $2-OCH_3$ | 3 | H | O | 148 - 150 |
| 1.87 | $CH_3$ | $2-OCH_2CH_2CH_3$ | 3 | H | O | 138 - 140 |
| 1.88 | $OCH_3$ | $2-OCH_2CH_2CH_3$ | 3 | H | O | |
| 1.89 | $CH_3$ | $4-COOC_2H_5$ | 3 | H | O | |
| 1.90 | $OCH_3$ | $4-COOC_2H_5$ | 3 | H | O | |

**Tabelle 2:**

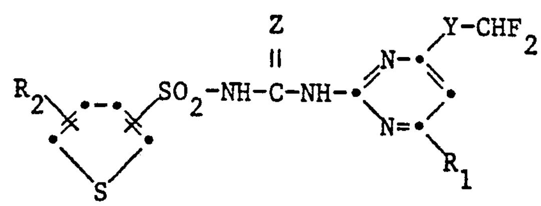

| Verb. Nr. | $R_1$ | $R_2$ | Stellung der Sulfonylgruppe | Z | Y | Smp. [°C] |
|---|---|---|---|---|---|---|
| 2.1 | $CH_3$ | $2-COOCH_3$ | 3 | O | O | 178 - 179 |
| 2.2 | $OCH_3$ | $2-COOCH_3$ | 3 | O | O | 171 - 174 |
| 2.3 | Cl | $2-COOCH_3$ | 3 | O | O | 119 - 120 |
| 2.4 | $-N(CH_3)_2$ | $2-COOCH_3$ | 3 | O | O | 232 |
| 2.5 | $C_2H_5$ | $2-COOCH_3$ | 3 | O | O | |
| 2.6 | $CF_3$ | $2-COOCH_3$ | 3 | O | O | |
| 2.7 | $-OCHF_2$ | $2-COOCH_3$ | 3 | O | O | 198 - 200 |

| | | | | | | |
|---|---|---|---|---|---|---|
| 2.8 | $CH_3$ | 4-$COOCH_3$ | 3 | O | O | 159 - 161 |
| 2.9 | $OCH_3$ | 4-$COOCH_3$ | 3 | O | O | 161 - 162 |
| 2.10 | Cl | 4-$COOCH_3$ | 3 | O | O | |
| 2.11 | -$N(CH_3)_2$ | 4-$COOCH_3$ | 3 | O | O | 225 - 227 |
| 2.12 | $C_2H_5$ | 4-$COOCH_3$ | 3 | O | O | |
| 2.13 | $CF_3$ | 4-$COOCH_3$ | 3 | O | O | |
| 2.14 | -$OCHF_2$ | 4-$COOCH_3$ | 3 | O | O | 178 - 179 |
| 2.15 | $CH_3$ | 2-CO-$OCH(CH_3)_2$ | 3 | O | O | 186 - 189 |
| 2.16 | $OCH_3$ | 2-CO-$OCH(CH_3)_2$ | 3 | O | O | |
| 2.17 | Cl | 2-CO-$OCH(CH_3)_2$ | 3 | O | O | |
| 2.18 | -$N(CH_3)_2$ | 2-CO-$OCH(CH_3)_2$ | 3 | O | O | |
| 2.19 | $OC_2H_5$ | 2-CO-$OCH(CH_3)_2$ | 3 | O | O | |
| 2.20 | $CF_3$ | 2-CO-$OCH(CH_3)_2$ | 3 | O | O | |
| 2.21 | -$OCHF_2$ | 2-CO-$OCH(CH_3)_2$ | 3 | O | O | |
| 2.22 | $CH_3$ | 2-CO-$OCH_2$-$CH=CH_2$ | 3 | O | O | |
| 2.23 | $OCH_3$ | 2-CO-$OCH_2$-$CH=CH_2$ | 3 | O | O | |
| 2.24 | Cl | 2-CO-$OCH_2$-$CH=CH_2$ | 3 | O | O | |
| 2.25 | -$N(CH_3)_2$ | 2-CO-$OCH_2$-$CH=CH_2$ | 3 | O | O | |
| 2.26 | $C_2H_5$ | 2-CO-$OCH_2$-$CH=CH_2$ | 3 | O | O | |
| 2.27 | $CF_3$ | 2-CO-$OCH_2$-$CH=CH_2$ | 3 | O | O | |
| 2.28 | -$OCHF_2$ | 2-CO-$OCH_2$-$CH=CH_2$ | 3 | O | O | |
| 2.29 | $CH_3$ | 5-Cl | 2 | O | O | |
| 2.30 | $OCH_3$ | 5-Cl | 2 | O | O | |
| 2.31 | Cl | 5-Cl | 2 | O | O | |
| 2.32 | -$N(CH_3)_2$ | 5-Cl | 2 | O | O | |
| 2.33 | $C_2H_5$ | 5-Cl | 2 | O | O | |
| 2.34 | $CF_3$ | 5-Cl | 2 | O | O | |
| 2.35 | -$OCHF_2$ | 5-Cl | 2 | O | O | |
| 2.36 | $CH_3$ | H | 2 | O | O | |
| 2.37 | $OCH_3$ | H | 2 | O | O | |
| 2.38 | Cl | H | 2 | O | O | |
| 2.39 | -$N(CH_3)_2$ | H | 2 | O | O | |
| 2.40 | $C_2H_5$ | H | 2 | O | O | |
| 2.41 | $CF_3$ | H | 2 | O | O | |
| 2.42 | -$OCHF_2$ | H | 2 | O | O | |
| 2.43 | $CH_3$ | H | 3 | O | O | |
| 2.44 | $OCH_3$ | H | 3 | O | O | |
| 2.45 | Cl | H | 3 | O | O | |
| 2.46 | -$N(CH_3)_2$ | H | 3 | O | O | |
| 2.47 | $C_2H_5$ | H | 3 | O | O | |
| 2.48 | $CF_3$ | H | 3 | O | O | |
| 2.49 | -$OCHF_2$ | H | 3 | O | O | |
| 2.50 | $CH_3$ | 2-$COOCH_3$ | 3 | S | O | |
| 2.51 | $OCH_3$ | 2-$COOCH_3$ | 3 | S | O | |
| 2.52 | Cl | 2-$COOCH_3$ | 3 | S | O | |
| 2.53 | -$N(CH_3)_2$ | 2-$COOCH_3$ | 3 | S | O | |
| 2.54 | $C_2H_5$ | 2-$COOCH_3$ | 3 | S | O | |
| 2.55 | $CF_3$ | 2-$COOCH_3$ | 3 | S | O | |
| 2.56 | -$OCHF_2$ | 2-$COOCH_3$ | 3 | S | O | |
| 2.57 | $CH_3$ | 4-$COOCH_3$ | 3 | S | O | |
| 2.58 | $OCH_3$ | 4-$COOCH_3$ | 3 | S | O | |
| 2.59 | Cl | 4-$COOCH_3$ | 3 | S | O | |
| 2.60 | -$N(CH_3)_2$ | 4-$COOCH_3$ | 3 | S | O | |
| 2.61 | $C_2H_5$ | 4-$COOCH_3$ | 3 | S | O | |
| 2.62 | $CF_3$ | 4-$COOCH_3$ | 3 | S | O | |
| 2.63 | -$OCHF_2$ | 4-$COOCH_3$ | 3 | S | O | |
| 2.64 | $CH_3$ | 3-$COOCH_3$ | 2 | S | O | |
| 2.65 | $OCH_3$ | 3-$COOCH_3$ | 2 | S | O | |
| 2.66 | Cl | 3-$COOCH_3$ | 2 | S | O | |
| 2.67 | -$N(CH_3)_2$ | 3-$COOCH_3$ | 2 | S | O | |
| 2.68 | $C_2H_5$ | 3-$COOCH_3$ | 2 | S | O | |
| 2.69 | $CF_3$ | 3-$COOCH_3$ | 2 | S | O | |
| 2.70 | -$OCHF_2$ | 3-$COOCH_3$ | 2 | S | O | |
| 2.71 | $CH_3$ | 3-$COOCH_3$ | 2 | O | S | |
| 2.72 | $OCH_3$ | 3-$COOCH_3$ | 2 | O | S | |

| | | | | | |
|---|---|---|---|---|---|
| 2.73 | Cl | 3-$COOCH_3$ | 2 | O | S |
| 2.74 | -$N(CH_3)_2$ | 3-$COOCH_3$ | 2 | O | S |
| 2.75 | $C_2H_5$ | 3-$COOCH_3$ | 2 | O | S |
| 2.76 | $CF_3$ | 3-$COOCH_3$ | 2 | O | S |
| 2.77 | -$OCHF_2$ | 3-$COOCH_3$ | 2 | O | S |
| 2.78 | $CH_3$ | 2-$COOCH_3$ | 3 | O | S |
| 2.79 | $OCH_3$ | 2-$COOCH_3$ | 3 | O | S |
| 2.80 | Cl | 2-$COOCH_3$ | 3 | O | S |
| 2.81 | -$N(CH_3)_2$ | 2-$COOCH_3$ | 3 | O | S |
| 2.82 | $C_2H_5$ | 2-$COOCH_3$ | 3 | O | S |
| 2.83 | $CF_3$ | 2-$COOCH_3$ | 3 | O | S |
| 2.84 | -$OCHF_2$ | 2-$COOCH_3$ | 3 | O | S |
| 2.85 | $CH_3$ | 4-$COOCH_3$ | 3 | O | S |
| 2.86 | $OCH_3$ | 4-$COOCH_3$ | 3 | O | S |
| 2.87 | Cl | 4-$COOCH_3$ | 3 | O | S |
| 2.88 | -$N(CH_3)_2$ | 4-$COOCH_3$ | 3 | O | S |
| 2.89 | $C_2H_5$ | 4-$COOCH_3$ | 3 | O | S |
| 2.90 | $CF_3$ | 4-$COOCH_3$ | 3 | O | S |
| 2.91 | -$OCHF_3$ | 4-$COOCH_3$ | 3 | O | S |
| 2.92 | $CH_3$ | 2-$SO_2$-$CH_3$ | 3 | O | O |
| 2.93 | $OCH_3$ | 2-$SO_2$-$CH_3$ | 3 | O | O |
| 2.94 | Cl | 2-$SO_2$-$CH_3$ | 3 | O | O |
| 2.95 | -$N(CH_3)_2$ | 2-$SO_2$-$CH_3$ | 3 | O | O |
| 2.96 | $C_2H_5$ | 2-$SO_2$-$CH_3$ | 3 | O | O |
| 2.97 | $CF_3$ | 2-$SO_2$-$CH_3$ | 3 | O | O |
| 2.98 | -$OCHF_2$ | 2-$SO_2$-$CH_3$ | 3 | O | O |
| 2.99 | $CH_3$ | 3-$SO_2$-$CH_3$ | 2 | O | O |
| 2.100 | $OCH_3$ | 3-$SO_2$-$CH_3$ | 2 | O | O |
| 2.101 | Cl | 3-$SO_2$-$CH_3$ | 2 | O | O |
| 2.102 | -$N(CH_3)_2$ | 3-$SO_2$-$CH_3$ | 2 | O | O |
| 2.103 | $C_2H_5$ | 3-$SO_2$-$CH_3$ | 2 | O | O |
| 2.104 | CF | 3-$SO_2$-$CH_3$ | 2 | O | O |
| 2.105 | -$OCHF_2$ | 3-$SO_2$-$CH_3$ | 2 | O | O |
| 2.106 | $CH_3$ | 4-$SO_2$-$CH_3$ | 3 | O | O |
| 2.107 | $OCH_3$ | 4-$SO_2$-$CH_3$ | 3 | O | O |
| 2.108 | Cl | 4-$SO_2$-$CH_3$ | 3 | O | O |
| 2.109 | -$N(CH_3)_2$ | 4-$SO_2$-$CH_3$ | 3 | O | O |
| 2.110 | $C_2H_5$ | 4-$SO_2$-$CH_3$ | 3 | O | O |
| 2.111 | $CF_3$ | 4-$SO_2$-$CH_3$ | 3 | O | O |
| 2.112 | $OCHF_2$ | 4-$SO_2$-$CH_3$ | 3 | O | O |
| 2.113 | $CH_3$ | 2-$SO_2$-$N(CH_3)_2$ | 3 | O | O |
| 2.114 | $OCH_3$ | 2-$SO_2$-$N(CH_3)_2$ | 3 | O | O |
| 2.115 | Cl | 2-$SO_2$-$N(CH_3)_2$ | 3 | O | O |
| 2.116 | -$N(CH_3)_2$ | 2-$SO_2$-$N(CH_3)_2$ | 3 | O | O |
| 2.117 | $C_2H_5$ | 2-$SO_2$-$N(CH_3)_2$ | 3 | O | O |
| 2.118 | $CF_3$ | 2-$SO_2$-$N(CH_3)_2$ | 3 | O | O |
| 2.119 | -$OCH_2$ | 2-$SO_2$-$N(CH_3)_2$ | 3 | O | O |
| 2.120 | $CH_3$ | 4-$SO_2$-$N(CH_3)_2$ | 3 | O | O |
| 2.121 | $OCH_3$ | 4-$SO_2$-$N(CH_3)_2$ | 3 | O | O |
| 2.122 | Cl | 4-$SO_2$-$N(CH_3)_2$ | 3 | O | O |
| 2.123 | -$N(CH_3)_2$ | 4-$SO_2$-$N(CH_3)_2$ | 3 | O | O |
| 2.124 | $C_2H_5$ | 4-$SO_2$-$N(CH_3)_2$ | 3 | O | O |
| 2.125 | $CF_3$ | 4-$SO_2$-$N(CH_3)_2$ | 3 | O | O |
| 2.126 | -$OCHF_2$ | 4-$SO_2$-$N(CH_3)_2$ | 3 | O | O |
| 2.127 | $CH_3$ | 3-$SO_2$-$N(CH_3)_2$ | 2 | O | O |
| 2.128 | $OCH_3$ | 3-$SO_2$-$N(CH_3)_2$ | 2 | O | O |
| 2.129 | Cl | 3-$SO_2$-$N(CH_3)_2$ | 2 | O | O |
| 2.130 | -$N(CH_3)_2$ | 3-$SO_2$-$N(CH_3)_3$ | 2 | O | O |
| 2.131 | $C_2H_5$ | 3-$SO_2$-$N(CH_3)_2$ | 2 | O | O |
| 2.132 | $CF_3$ | 3-$SO_2$-$N(CH_3)_2$ | 2 | O | O |
| 2.133 | -$OCHF_2$ | 3-$SO_2$-$N(CH_3)_2$ | 2 | O | O |
| 2.134 | $CH_3$ | 2-CO-$CH_3$ | 3 | O | O |
| 2.135 | $OCH_3$ | 2-CO-$CH_3$ | 3 | O | O |
| 2.136 | Cl | 2-CO-$CH_3$ | 3 | O | O |
| 2.137 | -$N(CH_3)_2$ | 2-CO-$CH_3$ | 3 | O | O |

| | | | | | | |
|---|---|---|---|---|---|---|
| 2.138 | $C_2H_5$ | $2\text{-}CO\text{-}CH_3$ | 3 | O | O | |
| 2.139 | $CF_3$ | $2\text{-}CO\text{-}CH_3$ | 3 | O | O | |
| 2.140 | $-OCHF_2$ | $2\text{-}CO\text{-}CH_3$ | 3 | O | O | |
| 2.141 | $CH_3$ | $2\text{-}COOC_2H_5$ | 3 | O | O | |
| 2.142 | $OCH_3$ | $2\text{-}COOC_2H_5$ | 3 | O | O | |
| 2.143 | Cl | $2\text{-}COOC_2H_5$ | 3 | O | O | |
| 2.144 | $-N(CH_3)_2$ | $2\text{-}COOC_2H_5$ | 3 | O | O | |
| 2.145 | $C_2H_5$ | $2\text{-}COOC_2H_5$ | 3 | O | O | |
| 2.146 | $CF_3$ | $2\text{-}COOC_2H_5$ | 3 | O | O | |
| 2.147 | $-OCHF_2$ | $2\text{-}COOC_2H_5$ | 3 | O | O | |
| 2.148 | $CH_3$ | $2\text{-}CO\text{-}OCH_2\text{-}C{\equiv}CH$ | 3 | O | O | 182 - 186 |
| 2.149 | $OCH_3$ | $2\text{-}CO\text{-}OCH_2\text{-}C{\equiv}CH$ | 3 | O | O | |
| 2.150 | Cl | $2\text{-}CO\text{-}OCH_2\text{-}C{\equiv}CH$ | 3 | O | O | |
| 2.151 | $-N(CH_3)_2$ | $2\text{-}CO\text{-}OCH_2\text{-}C{\equiv}CH$ | 3 | O | O | |
| 2.152 | $C_2H_5$ | $2\text{-}CO\text{-}OCH_2\text{-}C{\equiv}CH$ | 3 | O | O | |
| 2.153 | $CF_3$ | $2\text{-}CO\text{-}OCH_2{\equiv}CH$ | 3 | O | O | |
| 2.154 | $-OCHF_2$ | $2\text{-}CO\text{-}OCH_2{\equiv}CH$ | 3 | O | O | |
| 2.155 | $CH_3$ | $3\text{-}COOCH_3$ | 2 | O | O | |
| 2.156 | $OCH_3$ | $3\text{-}COOCH_3$ | 3 | O | O | |
| 2.157 | Cl | $3\text{-}COOCH_3$ | 2 | O | O | |
| 2.158 | $-N(CH_3)_2$ | $3\text{-}COOCH_3$ | 2 | O | O | |
| 2.159 | $C_2H_5$ | $3\text{-}COOCH_3$ | 2 | O | O | |
| 2.160 | $CF_3$ | $3\text{-}COOCH_3$ | 2 | O | O | |
| 2.161 | $-OCHF_2$ | $3\text{-}COOCH_3$ | 2 | O | O | |
| 2.162 | $CH_3$ | $3\text{-}COOC_2H_5$ | 2 | O | O | |
| 2.163 | $OCH_3$ | $3\text{-}COOC_2H_5$ | 2 | O | O | |
| 2.164 | Cl | $3\text{-}COOC_2H_5$ | 2 | O | O | |
| 2.165 | $-N(CH_3)_2$ | $3\text{-}COOC_2H_5$ | 2 | O | O | |
| 2.166 | $C_2H_5$ | $3\text{-}COOC_2H_5$ | 2 | O | O | |
| 2.167 | $CF_3$ | $3\text{-}COOC_2H_5$ | 2 | O | O | |
| 2.168 | $-OCHF_2$ | $3\text{-}COOC_2H_5$ | 2 | O | O | |
| 2.169 | $CH_3$ | $3\text{-}CO\text{-}OCH(CH_3)_2$ | 2 | O | O | |
| 2.170 | $OCH_3$ | $3\text{-}CO\text{-}OCH(CH_3)_2$ | 2 | O | O | |
| 2.171 | Cl | $3\text{-}CO\text{-}OCH(CH_3)_2$ | 2 | O | O | |
| 2.172 | $-N(CH_3)_2$ | $3\text{-}CO\text{-}OCH(CH_3)_2$ | 2 | O | O | |
| 2.173 | $C_2H_5$ | $3\text{-}CO\text{-}OCH(CH_3)_2$ | 2 | O | O | |
| 2.174 | $CF_3$ | $3\text{-}CO\text{-}OCH(CH_3)_2$ | 2 | O | O | |
| 2.175 | $-OCHF_2$ | $3\text{-}CO\text{-}OCH(CH_3)_2$ | 2 | O | O | |
| 2.176 | $CH_3$ | $3\text{-}CO\text{-}N(CH_3)_2$ | 2 | O | O | |
| 2.177 | $OCH_3$ | $3\text{-}CO\text{-}N(CH_3)_2$ | 2 | O | O | |
| 2.178 | Cl | $3\text{-}CO\text{-}N(CH_3)_2$ | 2 | O | O | |
| 2.179 | $-N(CH_3)_2$ | $3\text{-}CO\text{-}N(CH_3)_2$ | 2 | O | O | |
| 2.180 | $C_2H_5$ | $3\text{-}CO\text{-}N(CH_3)_2$ | 2 | O | O | |
| 2.181 | $CF_3$ | $3\text{-}CO\text{-}N(CH_3)_2$ | 2 | O | O | |
| 2.182 | $-OCHF_2$ | $3\text{-}CO\text{-}N(CH_3)_2$ | 2 | O | O | |
| 2.183 | $CH_3$ | $2\text{-}CO\text{-}N(CH_3)_2$ | 3 | O | O | |
| 2.184 | $OCH_3$ | $2\text{-}CO\text{-}N(CH_3)_2$ | 3 | O | O | |
| 2.185 | Cl | $2\text{-}CO\text{-}N(CH_3)_2$ | 3 | O | O | |
| 2.186 | $-N(CH_3)_2$ | $2\text{-}CO\text{-}N(CH_3)_2$ | 3 | O | O | |
| 2.187 | $C_2H_5$ | $2\text{-}CO\text{-}N(CH_3)_2$ | 3 | O | O | |
| 2.188 | $CF_3$ | $2\text{-}CO\text{-}N(CH_3)_2$ | 3 | O | O | |
| 2.189 | $-OCHF_2$ | $2\text{-}CO\text{-}N(CH_3)_2$ | 3 | O | O | |
| 2.190 | $CH_3$ | $4\text{-}CO\text{-}N(CH_3)_2$ | 3 | O | O | |
| 2.191 | $OCH_3$ | $4\text{-}CO\text{-}N(CH_3)_2$ | 3 | O | O | |
| 2.192 | Cl | $4\text{-}CO\text{-}N(CH_3)_2$ | 3 | O | O | |
| 2.193 | $-N(CH_3)_2$ | $4\text{-}CO\text{-}N(CH_3)_2$ | 3 | O | O | |
| 2.194 | $C_2H_5$ | $4\text{-}CO\text{-}N(CH_3)_2$ | 3 | O | O | |
| 2.195 | $CF_3$ | $4\text{-}CO\text{-}N(CH_3)_2$ | 3 | O | O | |
| 2.196 | $-OCHF_2$ | $4\text{-}CO\text{-}N(CH_3)_2$ | 3 | O | O | |
| 2.197 | $CH_3$ | $2\text{-}NO_2$ | 3 | O | O | |
| 2.198 | $OCH_3$ | $2\text{-}NO_2$ | 3 | O | O | |
| 2.199 | $OCHF_2$ | $2\text{-}NO_2$ | 3 | O | O | |
| 2.200 | $CH_3$ | $3\text{-}NO_2$ | 2 | O | O | |
| 2.201 | $OCH_3$ | $3\text{-}NO_2$ | 2 | O | O | |
| 2.202 | $OCHF_2$ | $3\text{-}NO_2$ | 2 | O | O | |

| | | | | | |
|---|---|---|---|---|---|
| 2.203 | $CH_3$ | 3-$NO_2$ | 4 | O | O |
| 2.204 | $OCH_3$ | 3-$NO_2$ | 4 | O | O |
| 2.205 | $OCHF_2$ | 3-$NO_2$ | 4 | O | O |
| 2.206 | $CH_3$ | 4-COO-$CH_2$-C≡CH | 3 | O | O |
| 2.207 | $OCH_3$ | 4-COO-$CH_2$-C≡CH | 3 | O | O |
| 2.208 | $OCH_3$ | 4-COO-$CH_2$-CH≡$CH_2$ | 3 | O | O |
| 2.209 | $CH_3$ | 4-$COOC_2H_5$ | 3 | O | O |
| 2.210 | $OCH_3$ | 4-$COOC_2H_5$ | 3 | O | O |

**Tabelle 3:**

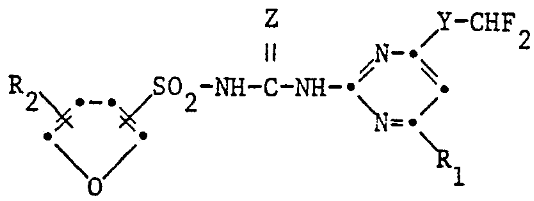

| Verb. Nr. | $R_1$ | $R_2$ | Stellung der Sulfonylgruppe | Z | Y | Smp. [°C] |
|---|---|---|---|---|---|---|
| 3.1 | $CH_3$ | 2-$COOCH_3$ | 3 | O | O | |
| 3.2 | $OCH_3$ | 2-$COOCH_3$ | 3 | O | O | |
| 3.3 | Cl | 2-$COOCH_3$ | 3 | O | O | |
| 3.4 | -$N(CH_3)_2$ | 2-$COOCH_3$ | 3 | O | O | |
| 3.5 | $C_2H_5$ | 2-$COOCH_3$ | 3 | O | O | |
| 3.6 | $CF_3$ | 2-$COOCH_3$ | 3 | O | O | |
| 3.7 | -$OCHF_2$ | 2-$COOCH_3$ | 3 | O | O | |
| 3.8 | $CH_3$ | 3-$COOCH_3$ | 2 | O | O | |
| 3.9 | $OCH_3$ | 3-$COOCH_3$ | 2 | O | O | |
| 3.10 | Cl | 3-$COOCH_3$ | 2 | O | O | |
| 3.11 | -$N(CH_3)_2$ | 3-$COOCH_3$ | 2 | O | O | |
| 3.12 | $C_2H_5$ | 3-$COOCH_3$ | 2 | O | O | |
| 3.13 | $CF_3$ | 3-$COOCH_3$ | 2 | O | O | |
| 3.14 | -$OCHF_2$ | 3-$COOCH_3$ | 2 | O | O | |
| 3.15 | $CH_3$ | 4-$COOCH_3$ | 3 | O | O | |
| 3.16 | $OCH_3$ | 4-$COOCH_3$ | 3 | O | O | |
| 3.17 | Cl | 4-$COOCH_3$ | 3 | O | O | |
| 3.18 | -$N(CH_3)_2$ | 4-$COOCH_3$ | 3 | O | O | |
| 3.19 | $C_2H_5$ | 4-$COOCH_3$ | 3 | O | O | |
| 3.20 | $CF_3$ | 4-$COOCH_3$ | 3 | O | O | |
| 3.21 | -$OCHF_2$ | 4-$COOCH_3$ | 3 | O | O | |
| 3.22 | $CH_3$ | 2-$COOCH_3$ | 3 | S | O | |
| 3.23 | $OCH_3$ | 2-$COOCH_3$ | 3 | S | O | |
| 3.24 | Cl | 2-$COOCH_3$ | 3 | S | O | |
| 3.25 | -$N(CH_3)_2$ | 2-$COOCH_3$ | 3 | S | O | |
| 3.26 | $C_2H_5$ | 2-$COOCH_3$ | 3 | S | O | |
| 3.27 | $CF_3$ | 2-$COOCH_3$ | 3 | S | O | |
| 3.28 | -$OCHF_2$ | 2-$COOCH_3$ | 3 | S | O | |

**Tabelle 4:**

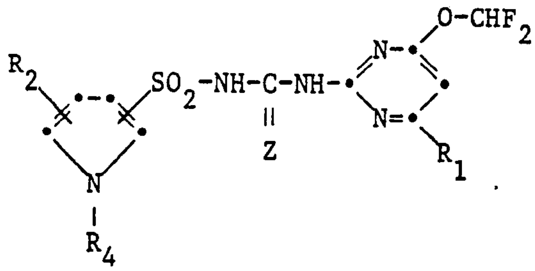

| Verb. Nr. | $R_1$ | $R_2$ | Stellung der Sulfonylgruppe | $R_4$ | Z | Smp. [°C] |
|---|---|---|---|---|---|---|
| 4.1 | $CH_3$ | H | 2 | H | O | 189 |
| 4.2 | $OCH_3$ | H | 2 | H | O | |
| 4.3 | Cl | H | 2 | H | O | |
| 4.4 | $-N(CH_3)_2$ | H | 2 | H | O | |
| 4.5 | $C_2H_5$ | H | 2 | H | O | |
| 4.6 | $CF_3$ | H | 2 | H | O | |
| 4.7 | $OCHF_2$ | H | 2 | H | O | |
| 4.8 | $CH_3$ | H | 3 | H | O | 173 - 174 |
| 4.9 | $OCH_3$ | H | 3 | H | O | |
| 4.10 | Cl | H | 3 | H | O | |
| 4.11 | $-N(CH_3)_2$ | H | 3 | H | O | |
| 4.12 | $C_2H_5$ | H | 3 | H | O | |
| 4.13 | $CF_3$ | H | 3 | H | O | |
| 4.14 | $-OCHF_3$ | H | 3 | H | O | |
| 4.15 | $CH_3$ | H | 3 | $CH_3$ | O | |
| 4.16 | $OCH_3$ | H | 3 | $CH_3$ | O | |
| 4.17 | Cl | H | 3 | $CH_3$ | O | |
| 4.18 | $-N(CH_3)_2$ | H | 3 | $CH_3$ | O | |
| 4.19 | $C_2H_5$ | H | 3 | $CH_3$ | O | |
| 4.20 | $CF_3$ | H | 3 | $CH_3$ | O | |
| 4.21 | $-OCHF_2$ | H | 3 | $CH_3$ | O | |
| 4.22 | $CH_3$ | H | 2 | $-(CH_2)_2-CN$ | O | |
| 4.23 | $OCH_3$ | H | 2 | $-(CH_2)_2-CN$ | O | |
| 4.24 | Cl | H | 2 | $-(CH_2)_2-CN$ | O | |
| 4.25 | $-N(CH_3)_2$ | H | 2 | $-(CH_2)_2-CN$ | O | |
| 4.26 | $C_2H_5$ | H | 2 | $-(CH_2)_2-CN$ | O | |
| 4.27 | $CF_3$ | H | 2 | $-(CH_2)_2-CN$ | O | |
| 4.28 | $-OCHF_2$ | H | 2 | $-(CH_2)_2-CN$ | O | |
| 4.29 | $CH_3$ | H | 2 | $CH_3$ | O | |
| 4.30 | $OCH_3$ | H | 2 | $CH_3$ | O | |
| 4.31 | Cl | H | 2 | $CH_3$ | O | |
| 4.32 | $N(CH_3)_2$ | H | 2 | $CH_3$ | O | |
| 4.33 | $CH_2CH_3$ | H | 2 | $CH_3$ | O | |
| 4.34 | $CF_3$ | H | 2 | $CH_3$ | O | |
| 4.35 | $OCHF_2$ | H | 2 | $CH_3$ | O | |
| 4.36 | $CH_3$ | H | 3 | $CH_3$ | O | |
| 4.37 | $OCH_3$ | H | 3 | $CH_3$ | O | |
| 4.38 | Cl | H | 3 | $CH_3$ | O | |
| 4.39 | $N(CH_3)_2$ | H | 3 | $CH_3$ | O | |
| 4.40 | $CH_2CH_3$ | H | 3 | $CH_3$ | O | |
| 4.41 | $CF_3$ | H | 3 | $CH_3$ | O | |
| 4.42 | $OCHF_2$ | H | 3 | $CH_3$ | O | |
| 4.43 | $CH_3$ | H | 3 | $CH_3$ | S | |
| 4.44 | $OCH_3$ | H | 3 | $CH_3$ | S | |
| 4.45 | Cl | H | 3 | $CH_3$ | S | |
| 4.46 | $N(CH_3)_2$ | H | 3 | $CH_3$ | S | |
| 4.47 | $CH_2CH_3$ | H | 3 | $CH_3$ | S | |
| 4.48 | $CF_3$ | H | 3 | $CH_3$ | S | |
| 4.49 | $OCHF_2$ | H | 3 | $CH_3$ | S | |

| | | | | | |
|---|---|---|---|---|---|
| 4.50 | $CH_3$ | 3-$COOCH_3$ | 2 | $CH_3$ | O |
| 4.51 | $OCH_3$ | 3-$COOCH_3$ | 2 | $CH_3$ | O |
| 4.52 | Cl | 3-$COOCH_3$ | 2 | $CH_3$ | O |
| 4.53 | $N(CH_3)_2$ | 3-$COOCH_3$ | 2 | $CH_3$ | O |
| 4.54 | $CH_2CH_3$ | 3-$COOCH_3$ | 2 | $CH_3$ | O |
| 4.55 | $CF_3$ | 3-$COOCH_3$ | 2 | $CH_3$ | O |
| 4.56 | $OCHF_2$ | 3-$COOCH_3$ | 2 | $CH_3$ | O |

**Formulierungsbeispiele**

**Beispiel 5:** Formulierungsbeispiele für Wirkstoffe der Formel 1 (% = Gewichtsprozent)

| a) Spritzpulver | a) | b) | c) |
|---|---|---|---|
| Wirkstoff | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther (7 - 8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

| d) Extruder-Granulat | a) | b) |
|---|---|---|
| Wirkstoff | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff | 40 % | 5 % |

| | | |
|---|---|---|
| Äthylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37-%-ige wässrige Formaldehyd-Lösung | 0,2 % | 0,2 % |
| Silikonöl in Form einer 75-%-igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) <u>Salzlösung</u>

| | |
|---|---|
| Wirkstoff | 5 % |
| Isopropylamin | 1 % |
| Octylphenolpolyäthylenglykoläther (78 Mol AeO) | 3 % |
| Wasser | 91 % |

**Biologische Beispiele**

**Beispiel 6:** Herbizidwirkung vor dem Auflaufen der Pflanzen

a) Kunststofftöpfe werden mit expandiertem Vermiculit (Dichte: 0.135 g/cm$^3$, Wasserabsorptionsvermögen: 0,565 1/1) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70 % gehalten. Während der Keimphase von 4 bis 5 Tagen werden die Töpfe zur Erhöhung der örtlichen Luftfeuchtigkeit mit lichtdurchlässigem Material abgedeckt und mit deionsiertem Wasser gegossen. Nach dem 5. Tag wird dem Gieswasser 0,5 % eines handelsüblichen Flüssigdüngers (®Greenzit) zugesetzt. 12 Tage nach der Aussaat wird der Versuch ausgewertet und die Wirkung auf die Versuchspflanzen nach dem folgenden Maßstab bewertet:

1: Pflanze nicht gekeimt oder total abgestorben
2 - 3: sehr starke Wirkung
4 - 6: mittlere Wirkung
7 - 8: schwache Wirkung
9: keine Wirkung (wie unbehandelte Kontrolle).

**pre-emergente Wirkung:**

Konzentration der Wirkstoffemulsion: 70,8 ppm

| Testpflanze | Nasturtium | Stellaria | Agrostis | Digitaria |
|---|---|---|---|---|
| Wirkstoff Nr. | | | | |
| 1.15 | 1 | 1 | 1 | 1 |
| 1.29 | 2 | 3 | 2 | 3 |
| 1.50 | 1 | 2 | 1 | 1 |
| 1.86 | 2 | 6 | 2 | 2 |
| 2.1 | 1 | 1 | 1 | 1 |
| 2.2 | 1 | 1 | 1 | 1 |
| 2.3 | 2 | 4 | 2 | 3 |
| 2.4 | 1 | 4 | 1 | 3 |
| 2.7 | 1 | 3 | 1 | 6 |
| 2.8 | 1 | 2 | 1 | 2 |
| 2.9 | 1 | 2 | 1 | 2 |
| 2.11 | 1 | 2 | 1 | 2 |
| 2.14 | 1 | 2 | 1 | 3 |

b) Im Gewächshaus werden Pflanzensamen in Blumentöpfe von 12 - 13 cm Durchmesser gesät. Unmittelbar

danach wird die Erdoberfläche mit einer wässrigen Dispersion oder Lösung der Wirkstoffe behandelt. Es werden Konzentrationen von 125 und 30 g Wirkstoffmengen pro Hektar angewendet. Die Töpfe werden dann im Gewächshaus bei einer Temperatur von 22 - 25° C und 50 - 70 % relativer Luftfeuchtigkeit gehalten. Nach 3 Wochen wird der Versuch ausgewertet. Die Wirkung auf die Versuchspflanzen nach dem oben angegebenen Maßstab bewertet.

**pre-emergent Wirkung**

| Wirkung<br>Aufwandmenge<br>gAs/ha | Verb. | | Verb. | | Verb. | | Verb. | |
|---|---|---|---|---|---|---|---|---|
| Testpflanze | 125 | 30 | 125 | 30 | 125 | 30 | 125 | 30 |
| Gerste | 8 | 9 | 2 | 4 | 2 | 6 | 7 | 9 |
| Weizen | 6 | 8 | 3 | 4 | 3 | 7 | 8 | 9 |
| Mais | 6 | 8 | 7 | 8 | 8 | 9 | 9 | 9 |
| Reis trocken | 3 | 6 | 1 | 1 | 1 | 2 | 6 | 7 |
| Alopecurus myos. | 5 | 6 | 2 | 3 | 1 | 2 | 2 | 4 |
| Echinochloa c.g. | 8 | 9 | 3 | 4 | 4 | 7 | 9 | 9 |
| Rottboellia ex. | 8 | 9 | 6 | 7 | 5 | 7 | 6 | 9 |
| Cyperus escul. | 4 | 8 | 1 | 4 | 1 | 2 | 3 | 9 |
| Soja | 8 | 8 | 8 | 9 | 5 | 7 | 7 | 9 |
| Baumwolle | 4 | 7 | 4 | 5 | 3 | 5 | 9 | 9 |
| Zuckerrübe | 2 | 2 | 2 | 3 | 1 | 1 | 2 | 4 |
| Abutilon | 2 | 4 | 5 | 6 | 2 | 4 | 7 | 7 |
| Xanthium Sp. | 7 | 9 | 4 | 5 | 3 | 4 | 6 | 9 |
| Amaranthus ret. | 3 | 3 | 2 | 3 | 2 | 2 | 2 | 2 |
| Chenopodium Sp. | 4 | 6 | 5 | 6 | 2 | 2 | 3 | 9 |
| Solanum nigrum | 5 | 8 | 3 | 4 | 2 | 3 | 3 | 9 |
| Ipomoea | 4 | 9 | 6 | 8 | 4 | 6 | 3 | 9 |
| Sinapis | 2 | 3 | 3 | 4 | 1 | 1 | 3 | 9 |
| Stellaria | 3 | 4 | 3 | 4 | 2 | 2 | 2 | 2 |
| Chrysanthe. leuc. | 2 | 3 | 2 | 2 | 1 | 1 | 2 | 9 |
| Galium aparine | 3 | 5 | 3 | 3 | 1 | 2 | 2 | 5 |
| Viola tricolor | 2 | 2 | 3 | 3 | 1 | 2 | 3 | 9 |
| Veronica Sp. | 1 | 2 | 1 | 1 | 1 | 1 | 2 | 2 |

**Beispiel 7:** Nachweis der Herbizidwirkung nach dem Auflaufen der Pflanzen (Kontaktwirkung)

Eine Anzahl Unkräuter und Kulturpflanzen, sowohl monocotyle wie dicotyle, wurden nach dem Auflaufen, im 4- bis 6-Blattstadium mit einer wässrigen Wirkstoffdispersion in Dosierungen von 125 und 30 g AS/ha gespritzt und dann bei 24° bis 26° C und 45 - 60 % relativer Luftfeuchtigkeit gehalten. 15 Tage nach der Behandlung wird der Versuch ausgewertet und nach dem gleichen Maßstab wie im pre-emergenten Versuch bewertet.

**post-emergente Wirkung**

| Wirkung<br>Aufwandmenge<br>g As/ha | Verb. Nr. 2.2 | | Verb. Nr. 2.9 | |
|---|---|---|---|---|
| Testpflanze | 125 | 30 | 125 | 30 |
| Weizen | 8 | 9 | 7 | 8 |
| Mais | 8 | 9 | 8 | 9 |
| Reis trocken | 8 | 9 | 5 | 7 |
| Alopecurus myos. | 6 | 7 | 4 | 7 |
| Echinochloa c.g. | 8 | 9 | 6 | 7 |
| Cyperus escul. | 4 | 6 | 3 | 4 |
| Soja | 6 | 7 | 5 | 7 |
| Baumwolle | 5 | 6 | 8 | 8 |
| Zuckerrübe | 2 | 2 | 2 | 2 |

| | | | | |
|---|---|---|---|---|
| Abutilon | 3 | 4 | 2 | 3 |
| Xanthium Sp. | 2 | 2 | 2 | 2 |
| Chenopodium Sp. | 5 | 6 | 3 | 5 |
| Ipomoea | 4 | 7 | 4 | 5 |
| Sinapis | 2 | 2 | 3 | 3 |
| Galium aparine | 4 | 5 | 4 | 6 |
| Viola tricolor | 2 | 3 | 2 | 4 |

**Beispiel 8:** Nachweis der Wuchshemmung bei tropischen Bodendeckern-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21°C bei Nach gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20 % des Neuzuwachses bei unbehandelten Kontrollpflanzen) ohne dass dabei die Versuchspflanzen geschädigt werden.

**Patentansprüche** für die Vertragsstaaten: BE, CH, DE, FR, GB, IT, LI, NL, SE

1. N-Heterocyclosulfonyl-N'-pyrimidinyl- und -triazinylharnstoffe der allgemeinen Formel

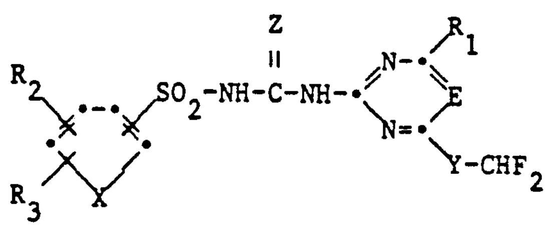

(I)

worin

X Sauerstoff, Schwefel, $-N(R_4)-$ oder $-C(R_5)=$

Y Sauerstoff oder Schwefel,
Z Sauerstoff oder Schefel
E Stickstoff oder -CH=,
$R_1$ Wasserstoff, $C_1$-$C_4$-Alkyl, $C_1$-$C_4$-Halogenalkyl, $C_1$-$C_4$-Halogenalkoxy, $C_1$-$C_4$-Alkoxy, Halogen, $-NR_6R_7$ oder Alkoxyalkyl mit höchstens 4 Kohlenstoffatomen,
$R_2$ Wasserstoff, $C_1$-$C_3$-Alkyl, $C_1$-$C_3$-Halogenalkyl, Halogen, Nitro, $C_1$-$C_3$-Alkoxy, $-C(=W)-R_8$, $-SO_2-NR_6R_7$, $-SO_n-C_1$-$C_3$-Alkyl oder $-CO-R_9$,
$R_3$ Wasserstoff, Halogen, $C_1$-$C_3$-Alkyl, Methoxy, Nitro oder Trifluormethyl,
$R_4$ Wasserstoff, $C_1$-$C_4$-Alkyl, Cyanoalkyl mit höchstens 4 Kohlenstoffatomen, $C_3$-$C_6$-Cycloalkyl, Benzyl, $-CO-C_1$-$C_4$-Alkoxy, $-CO-NR_6R_7$ oder gegebenenfalls durch 1 bis 3 Halogenatome substituiertes $-CO-C_1$-$C_4$-Alkyl,
$R_5$ Wasserstoff, Nitro, Fluor, Chlor, Brom, Methyl, Trifluormethyl, $-SO_n-C_1$-$C_3$-Alkyl, $-CO-C_1$-$C_4$-Alkoxy oder $C_1$-$C_3$-Alkoxy,
$R_6$ Wasserstoff, $C_1$-$C_6$-Alkyl, Cyanoalkyl mit hochstens 4 Kohlenstoffatomen, Methoxy oder Äthoxy,
$R_7$ Wasserstoff, $C_1$-$C_6$-Alkyl oder $C_3$-$C_6$-Alkenyl oder
$R_6$ und $R_7$ zusammen mit dem sie bindenden Stickstoffatom einen 5- bis 6-gliedrigen gesättigten, gegebenenfalls ein Sauerstoff- oder Schwefelatom als Ringglied enthaltenden, Heterocyclus,
$R_8$ Wasserstoff, $C_1$-$C_6$-Alkyl, $C_1$-$C_4$-Halogenalkyl, $C_3$-$C_6$-Cycloalkyl, $C_4$-$C_7$-Cycloalkylalkyl oder Alkoxyalkyl mit höchstens 4 Kohlenstoffatomen,
$R_9$ $C_1$-$C_6$-Alkoxy, $C_3$-$C_6$-Alkenyloxy, $C_3$-$C_6$-Alkinyloxy, $C_2$-$C_6$-Halogenalkoxy, $C_1$-$C_4$-Cyanoalkoxy, $C_1$-$C_6$-Alkylthio, $C_3$-$C_6$-Alkenylthio, $C_3$-$C_6$-Alkinylthio, $C_5$-$C_6$-Cycloalkoxy, $C_4$-$C_7$-Cycloalkylalkoxy, $-NR_6R_7$ oder Alkoxyalkoxy mit höchstens 6 Kohlenstoffatomen
W Sauerstoff oder $=N-O-R_{10}$, worin $R_{10}$ für Wasserstoff, $C_1$-$C_6$-Alkyl oder $C_3$-$C_6$-Alkenyl steht, und

n eine Zahl von Null bis zwei bedeuten, sowie die Salze dieser Verbindungen.

2. Verbindungen gemäss Anspruch 1, dadurch gekennzeichnet, dass Z Sauerstoff bedeutet.

3. Verbindungen gemäss Anspruch 1, dadurch gekennzeichnet, dass Y Sauerstoff bedeutet.

4. Verbindungen gemäss Anspruch 1, dadurch gekennzeichnet, dass X für Schwefel, $-NR_4-$ oder $-CR_5=N-$ steht.

5. Verbindungen gemäss Anspruch 1, dadurch gekennzeichnet, dass E für die Methinbrücke $-CH=$ steht.

6. Verbindungen gemäss Anspruch 1, dadurch gekennzeichnet, dass $R_1$ für Chlor, Dimethylamino, Trifluormethyl, Fluormethyl, Methoxy, Difluormethoxy, Methyl oder Äthyl steht.

7. Verbindungen gemäss Anspruch 1, dadurch gekennzeichnet, dass $R_3$ Wasserstoff bedeutet.

8. Verbindungen gemäss Anspruch 1, dadurch gekennzeichnet, dass der Rest $R_2$ sich in Nachbarstellung zur Sulfonylgruppe befindet.

9. Verbindungen gemäss Anspruch 8, dadurch gekennzeichnet, dass $R_2$ für Wasserstoff, Fluor, Chlor, Methoxy, Acetyl, $-SO_2-CH_3$, $-COOCH_2-CH=CH_2$, $-COOCH_2-C\equiv CH$, $-SO_2-N(CH_3)_2$ oder $-CO-C_1-C_4$-Alkoxy steht.

10. Verbindungen gemäss Anspruch 1, dadurch gekennzeichnet, dass Y und Z Sauerstoff, E die Methinbrücke, R Chlor, Dimethylamino, Trifluormethyl, Fluormethyl, Methoxy, Difluormethoxy, Methyl oder Äthyl bedeuten, $R_2$ sich in Nachbarstellung zur Sulfonylgruppe befindet und für Wasserstoff, Fluor, Chlor, Methoxy, Acetyl, $SO_2-CH_3$, $-COOCH_2-CH=CH_2$, $-COOCH_2-C\equiv CH$, $-SO_2-N(CH_3)_2$ oder $-CO-C_1-C_4$-Alkoxy steht und $R_3$ Wasserstoff bedeutet.

11. Verbindungen gemäss Anspurch 1, dadurch gekennzeichnet, dass X für ein Schwefelatom steht, Y und Z Sauerstoff, E die Methinbrücke, $R_1$ Chlor, Dimethylamino, Trifluormethyl, Fluormethyl, Methoxy, Difluormethoxy, Methyl oder Äthyl bedeuten, $R_2$ sich in Nachbarstellung zur Sulfonylgruppe befindet und für Wasserstoff, Fluor, Chlor, Methoxy, Acetyl, $-SO_2-CH_3$, $-COOCH_2-CH=CH_2$, $-COOCH_2-C=CH$, $-SO_2-N(CH_3)_2$ oder $-CO-C_1-C_4$-Alkoxy steht und $R_3$ Wasserstoff bedeutet.

12. Verbindungen gemäss Anspruch 1, dadurch gekennzeichnet, dass X für Schwefel oder $-CH=N-$ steht, Y und Z Sauerstoff, E die Methinbrücke, $R_1$ Chlor, Dimethylamino, Fluormethyl, Methoxy, Difluormethoxy, Methyl oder Äthyl bedeuten, $R_2$ sich in Nachbarstellung zur Sulfonylgruppe befindet und für Wasserstoff, Chlor, $-COOCH_2-CH=CH_2$, $-COOCH_2-C\equiv CH$ oder $-CO-C_1-C_4$-Alkoxy steht und $R_3$ Wasserstoff bedeutet.

13. Verbindungen gemäss Anspruch 1, dadurch gekennzeichnet, dass X für $-CR_5=N-$ steht, Y und Z Sauerstoff, E die Methinbrücke, $R_1$ Chlor, Dimethylamino, Trifluormethyl, Fluormethyl, Methoxy, Difluormethoxy, Methyl oder Äthyl bedeuten, $R_2$ sich in Nachbarstellung zur Sulfonylgruppe befindet und für Wasserstoff, Fluor, Chlor, Methoxy, Acetyl, $-SO_2-CH_3$, $-COOCH_2-CH=CH_2$, $-COOCH_2-C\equiv CH$, $-SO_2-N(CH_3)_2$ oder $-CO-C_1-C_4$-Alkoxy steht und $R_3$ Wasserstoff bedeutet.

14. N-(2-Methoxycarbonyl-3-thienylsulfonyl)-N'-(4-difluormethoxy-6-methylpyrimidin-2-yl)-harnstoff gemäss Anspruch 1.

15. N-(4-Methoxycarbonyl-3-thienylsulfonyl)-N'-(4-difluormethoxy-6-methylpyrimidin-2-yl)-harnstoff gemäss Anspruch 1.

16. N-(2-Chlor-3-pyridinylsulfonyl)-N'-(4-difluormethoxy-6-methylpyrimidin-2-yl)-harnstoff gemäss Anspruch 1.

17. Verfahren zur Herstellung der Verbindungen der Formel 1, gemäss Anspruch 1, dadurch gekennzeichnet, dass man entweder

a) ein Heterocyclosulfonamid der Formel

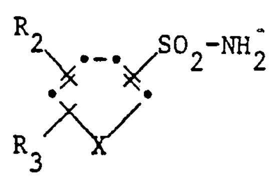

(II),

worin $R_2$, $R_3$ und X die unter Formel I gegebene Bedeutung haben, in Gegenwart einer Base mit einem N-Pyrimidinyl oder -triazinylcarbamat der Formel III

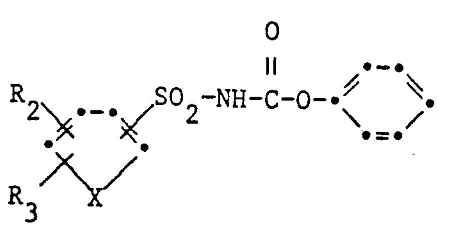

(VII)

worin E, $R_1$, Y und Z die unter Formel I gegebene Bedeutung haben oder

b) ein Heterocyclosulfonylisocyanat oder -isothiocyanat der Formel IV

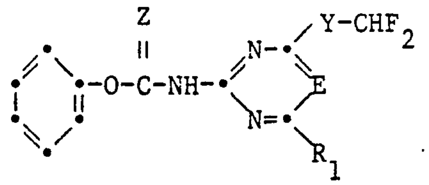

(III),

worin $R_2$, $R_3$, X und Z die unter Formel I gegebene Bedeutung haben, gegebenenfalls in Gegenwart einer Base, mit einem Aminopyrimidin oder -triazin der Formel V

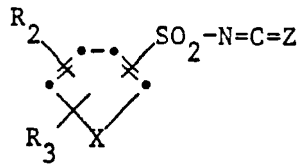

(IV)

worin E, $R_1$ und Y die unter Formel I gegebene Bedeutung haben oder

c) ein Heterocyclosulfonamid der oben angegebenen Formel II gegebenenfalls in Gegenwart einer Base mit einem Isocyanatoder Isothiocyanat der Formel VI

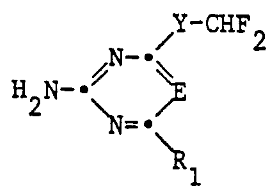

(V),

worin E, $R_1$, Y und Z die unter Formel I gegebene Bedeutung haben oder

d) ein N-Heterocyclosulfonylcarbamat der Formel VII

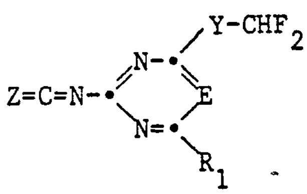

(VI),

worin $R_2$, $R_3$ und X die unter Formel I gegebene Bedeutung haben, mit einem Aminopyrimidin oder -triazin der oben angegebenen Formel V umsetzt und gegebenenfalls im die Salze überführt indem man einen Sulfonylharnstoff der Formel I mit einem Amin, einem Alkalimetall- oder Erdalkalimetallhydroxid oder einer quaternären Ammoniumbase umsetzt.

18. Ein herbizides und den Pflanzenwuchs hemmendes Mittel, dadurch gekennzeichnet, dass es neben Träger- und/oder anderen Zuschlagstoffen als Wirkstoff mindestens einen N-Heterocyclosulfonyl-N'-pyrimidinyloder triazinylharnstoff der Formel I, Anspruch 1, enthält.

19. Die Verwendung der N-Heterocyclosulfonyl-N'-pyrimidinyl- und triazinylharnstoffe der Formel I, Anspruch 1, oder sie enthaltender Mittel zur Bekämpfung unerwünschten Pflanzenwachstums.

20. Die Verwendung der Verbindungen der Formel I, Anspruch 1, oder sie enthaltender Mittel zur Regulierung des Pflanzenwachstums.

21. Die Verwendung der Verbindungen der Formel I, oder sie enthaltender Mittel, gemäss Anspruch 19, zur selektiven pre- oder post-emergenten Bekämpfung von Unkräutern in Nutzpflanzenkulturen.

22. Die Verwendung gemäss Anspurch 21 zur Bekämpfung von Unkräutern in Soja-Kulturen.

23. N-(4-Methoxycarbonyl-3-thienylsulfonyl)-N'-(4-difluormethoxy-6-methoxypyrimidin-2-yl)-harnstoff gemäss Anspruch 1.

**Patentansprüche** für den Vertragsstaat: AT

1. Ein herbizides und den Pflanzenwuchs hemmendes Mittel, dadurch gekennzeichnet, dass es neben Träger- und/oder anderen Zuschlagstoffen als Wirkstoff mindestens einen N-Heterocyclosulfonyl-N'-pyrimidinyloder triazinylharnstoff der Formel

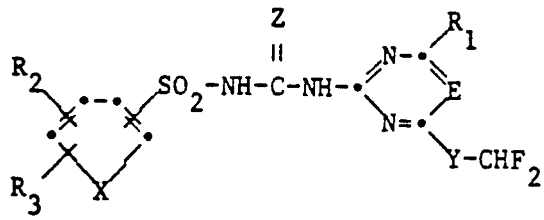

(I)

oder ein Salz dieser Verbindungen enthält, worin

| | |
|---|---|
| X | Sauerstoff, Schwefel, $\underset{R_4}{\underset{\vert}{-N-}}$ oder $\underset{R_5}{\underset{\vert}{-CN=}}$ |
| Y | Sauerstoff oder Schwefel, |
| Z | Sauerstoff oder Schefel |
| E | Stickstoff oder -CH=, |
| $R_1$ | Wasserstoff, $C_1$-$C_4$-Alkyl $C_1$-$C_4$-Halogenalkyl $C_1$-$C_4$-Halogenalkoxy, $C_1$-$C_4$-Alkoxy, Halogen, $-NR_6R_7$ oder Alkoxyalkyl mit höchstens 4 Kohlenstoffatomen, |
| $R_2$ | Wasserstoff, $C_1$-$C_3$-Alkyl, $C_1$-$C_3$-Halogenalkyl, Halogen, Nitro, $C_1$-$C_3$-Alkoxy, $\underset{W}{\underset{\Vert}{-C-R_8}}$, $-SO_2-NR_6R_7$, $-SO_n$-$C_1$-$C_3$-Alkyl oder $-CO-R_9$, |
| $R_3$ | Wasserstoff, Halogen, $C_1$-$C_3$-Alkyl, Methoxy, Nitro oder Trifluormethyl, |
| $R_4$ | Wasserstoff, $C_1$-$C_4$-Alkyl Cyanoalkyl mit höchstens 4 Kohlenstoffatomen, $C_3$-$C_6$-Cycloalkyl, Benzyl, -CO-$C_1$-$C_4$-Alkoxy, $-CO-NR_6R_7$ oder gegebenenfalls durch 1 bis 3 Halogenatome substituiertes -CO-$C_1$-$C_4$-Alkyl, |
| $R_5$ | Wasserstoff Nitro, Fluor, Chlor, Brom, Methyl, Trifluormethyl, $-SO_n$-$C_1$-$C_3$-Alkyl, -CO-$C_1$-$C_4$-Alkoxy oder $C_1$-$C_3$-Alkoxy, |
| $R_6$ | Wasserstoff, $C_1$-$C_6$-Alkyl, Cyanoalkyl mit hochstens 4 Kohlenstoffatomen, Methoxy oder Äthoxy, |
| $R_7$ | Wasserstoff, $C_1$-$C_6$-Alkyl oder $C_3$-$C_6$-Alkenyl oder |
| $R_6$ und $R_7$ | zusammen mit dem sie bindenden Stickstoffatom einen 5- bis 6-gliedrigen gesättigten, gegebenenfalls ein Sauerstoff- oder Schwefelatom als Ringglied enthaltenden, Heterocyclus, |
| $R_8$ | Wasserstoff, $C_1$-$C_6$-Alkyl, $C_1$-$C_4$-Halogenalkyl, $C_3$-$C_6$-Cycloalkyl, $C_4$-$C_7$-Cycloalkylalkyl oder Alkoxyalkyl mit höchstens 4 Kohlenstoffatomen, |
| $R_9$ | $C_1$-$C_6$-Alkoxy, $C_3$-$C_6$-Alkenyloxy, $C_3$-$C_6$-Alkinyloxy, $C_2$-$C_6$-Halogenalkoxy, $C_1$-C -Cyanoalkoxy, $C_1$-$C_6$-Alkylthio, $C_3$-$C_6$-Alkenylthio, $C_3$-$C_6$-Alkinylthio, $C_5$-$C_6$-Cycloalkoxy, $C_4$-$C_7$-Cycloalkylalkoxy, $-NR_6R_7$ oder Alkoxyalkoxy mit höchstens 6 Kohlenstoffatomen, |
| W | Sauerstoff oder $=N-O-R_{10}$, worin $R_{10}$ für Wasserstoff, $C_1$-$C_6$-Alkyl oder $C_3$-$C_6$-Alkenyl steht, und |
| n | eine Zahl von Null bis zwei bedeuten. |

2. Mittel gemäss Anspruch 1, dadurch gekennzeichnet, dass Z Sauerstoff bedeutet.

3. Mittel gemäss Anspruch 1, dadurch gekennzeichnet, dass Y Sauerstoff bedeutet.

4. Mittel gemäss Anspruch 1, dadurch gekennzeichnet, dass X für Schwefel, $-NR_4$- oder $-CR_5=N$- steht.

5. Mittel gemäss Anspruch 1, dadurch gekennzeichnet, dass E für die Methinbrücke -CH= steht.

6. Mittel gemäss Anspruch 1, dadurch gekennzeichnet, dass $R_1$ für Chlor, Dimethylamino, Trifluormethyl, Fluormethyl, Methoxy, Difluormethoxy, Methyl oder Äthyl steht.

7. Mittel gemäss Anspruch 1 dadurch gekennzeichnet, dass R Wasserstoff bedeutet.

8. Mittel gemäss Anspruch 1, dadurch gekennzeichnet, dass der Rest $R_2$ sich in Nachbarstellung zur Sulfonylgruppe befindet.

9. Mittel gemäss Anspruch 8, dadurch gekennzeichnet, dass R für Wasserstoff, Fluor, Chlor, Methoxy, Acetyl, $-SO_2-CH_3$, $-COOCH_2-CH=CH_2$, $-COOCH_2-C\equiv CH$, $-SO_2-N(CH_3)_2$ oder -CO-$C_1$-$C_4$-Alkoxy steht.

10. Mittel gemäss Anspruch 1, dadurch gekennzeichnet, dass Y und Z Sauerstoff, E die Methinbrücke, $R_1$ Chlor, Dimethylamino, Trifluormethyl, Fluormethyl, Methoxy, Difluormethoxy, Methyl oder Äthyl bedeuten, $R_2$ sich in Nachbarstellung zur Sulfonylgruppe befindet und für Wasserstoff, Fluor, Chlor, Methoxy, Acetyl, $SO_2$-$C_3$, $-COOCH_2-CH=CH_2$, $-COOCH_2-C\equiv CH$, $-SO_2-N(CH_3)_2$ oder -CO-$C_1$-$C_4$-Alkoxy steht und $R_3$ Wasserstoff bedeutet.

11. Mittel gemäss Anspruch 1, dadurch gekennzeichnet, dass X für ein Schwefelatom steht, Y und Z Sauerstoff, E die Methinbrücke, $R_1$ Chlor, Dimethylamino, Trifluormethyl, Fluormethyl, Methoxy, Difluormethoxy, Methyl oder Äthyl bedeuten, $R_2$ sich in Nachbarstellung zur Sulfonylgruppe befindet und für

Wasserstoff, Fluor, Chlor, Methoxy, Acetyl, $-SO_2-CH_3$, $-COOCH_2-CH=CH_2$, $-COOCH_2-C\equiv CH$, $-SO_2-N(CH_3)_2$ oder $-CO-C_1-C_4$-Alkoxy steht und $R_3$ Wasserstoff bedeutet.

12. Mittel gemäss Anspruch 1, dadurch gekennzeichnet, dass X für Schwefel oder $-CH=N-$ steht, Y und Z Sauerstoff, E die Methinbrücke, $R_1$ Chlor, Dimethylamino, Fluormethyl, Methoxy, Difluormethoxy, Methyl oder Äthyl bedeuten, $R_2$ sich in Nachbarstellung zur Sulfonylgruppe befindet und für Wasserstoff, Chlor, $-COOCH_2-CH=CH_2$, $-COOCH_2-C\equiv CH$ oder $-CO-C_1-C_4$-Alkoxy steht und $R_3$ Wasserstoff bedeutet.

13. Mittel gemäss Anspruch 1, dadurch gekennzeichnet, dass X für $-CR_5=N-$ steht, Y und Z Sauerstoff, E die Methinbrücke, $R_1$ Chlor, Dimethylamino, Trifluormethyl, Fluormethyl, Methoxy, Difluormethoxy, Methyl oder Äthyl bedeuten, $R_2$ sich in Nachbarstellung zur Sulfonylgruppe befindet und für Wasserstoff, Fluor, Chlor, Methoxy, Acetyl, $-SO_2-CH_3$, $-COOCH_2-CH=CH_2$, $-COOCH_2-C\equiv CH$, $-SO_2-N(CH_3)_2$ oder $-CO-C_1-C_4$-Alkoxy steht und $R_3$ Wasserstoff bedeutet.

14. Mittel gemäss Anspruch 1, dadurch gekennzeichnet, dass es als Wirkstoff N-(2-(Methoxycarbonyl-3-thienylsulfonyl)-N'-(4-difluormethoxy-6-methylpyrimidin-2-yl)-harnstoff enthält.

15. Mittel gemäss Anspruch 1, dadurch gekennzeichnet, dass es als Wirkstoff N-(4-Methoxycarbonyl-3-thienylsulfonyl)-N'-(4-difluormethoxy-6-methylpyrimidin-2-yl)-harnstoff enthält.

16. Mittel gemäss Anspruch 1, dadurch gekennzeichnet, dass es als Wirkstoff N-(2-Chlor-3-pyridinylsulfonyl)-N'-(4-difluormethoxy-6-methylpyrimidin-2-yl)-harnstoff enthält.

17. Verfahren zur Herstellung der Verbindungen der Formel I, gemäss Anspruch 1, dadurch gekennzeichnet, dass man entweder

a) ein Heterocyclosulfonamid der Formel II

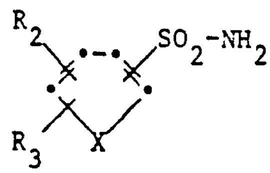 (II),

worin $R_2$, $R_3$ und X die unter Formel I gegebene Bedeutung haben in Gegenwart einer Base mit einem N-Pyrimidinyl oder -triazinylcarbamat der Formel III

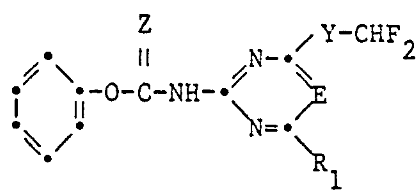 (III),

worin E, $R_1$, Y und Z die unter Formel I gegebene Bedeutung haben oder

b) ein Heterocyclosulfonylisocyanat oder -isothiocyanat der Formel IV

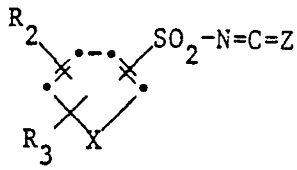 (IV)

worin $R_2$, $R_3$, X und Z die unter Formel I gegebene Bedeutung haben, gegebenenfalls in Gegenwart einer Base, mit einem Aminopyrimidin oder -triazin der Formel V

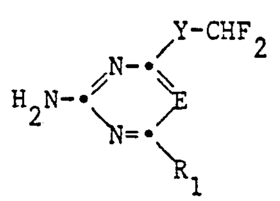 (V),

worin E, $R_1$ und Y die unter Formel I gegebene Bedeutung haben oder

c) ein Heterocyclosulfonamid der oben angegebenen Formel II gegebenenfalls in Gegenwart einer Base mit einem Isocyanatoder Isothiocyanat der Formel VI

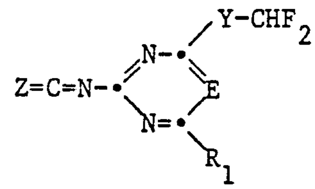

(VI),

worin E, $R_1$, Y und Z die unter Formel I gegebene Bedeutung haben oder

d) ein N-Heterocyclosulfonylcarbamat der Formel VII

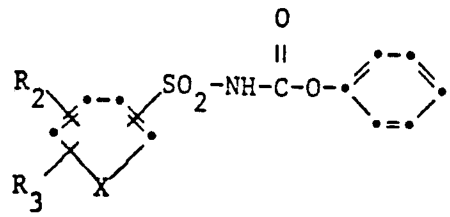

(VII)

worin $R_2$, $R_3$ und X die unter Formel I gegebene Bedeutung haben, mit einem Aminopyrimidin oder -triazin der oben angegebenen Formel V umsetzt und gegebenenfalls im die Salze überführt indem man einen Sulfonylharnstoff der Formel I mit einem Amin, einem Alkalimetall- oder Erdalkalimetallhydroxid oder einer quaternären Ammoniumbase umsetzt.

18. Die Verwendung der N-Heterocyclosulfonyl-N'-pyrimidinyl- und triazinylharnstoffe der Formel I, Anspruch 1, oder sie enthaltender Mittel zur Bekämpfung unerwünschten Pflanzenwachstums.

19. Die Verwendung der Verbindungen der Formel I, Anspruch 1, oder sie enthaltender Mittel zur Regulierung des Pflanzenwachstums.

20. Die Verwendung der Verbindungen der Formel I, oder sie enthaltender Mittel, gemäss Anspruch 18, zur selektiven pre- oder post-emergenten Bekämpfung von Unkräutern in Nutzpflanzenkulturen.

21. Die Verwendung gemäss Anspruch 20 zur Bekämpfung von Unkräutern in Soja-Kulturen.

22. Mittel gemäss Anspruch 1, dadurch gekennzeichnet, dass es als Wirkstoff N-(4-Methoxycarbonyl-3-thienylsulfonyl)-N'-(4-difluormethoxy-6-methoxypyrimidin-2-yl)-harnstoff enthält.

**Claims** for the Contracting States: BE, CH, DE, FR, GB, IT, LI, NL, SE

1. N-heterocyclosulfonyl-N'-pyrimidinylureas and N-heterocyclosulfonyl-N'-triazinylureas of the general formula I

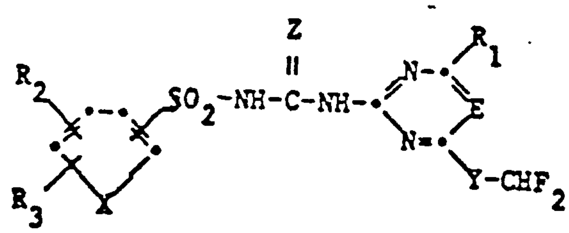

(I)

wherein

X is oxygen, sulfur, $-\underset{|}{N}-$ or $-\underset{|}{C}=N-$, with $R_4$ and $R_5$ respectively

Y is oxygen or sulfur,

Z is oxygen or sulfur,

E is nitrogen or -CH=,

$R_1$ is hydrogen, $C_1$-$C_4$alkyl, $C_1$-$C_4$haloalkyl, $C_1$-$C_4$haloalkoxy, $C_1$-$C_4$alkoxy, halogen, $-NR_6R_7$ or alkoxyalkyl containing not more than 4 carbon atoms,

$R_2$ is hydrogen, $C_1$-$C_3$alkyl, $C_1$-$C_3$haloalkyl, halogen, nitro, $C_1$-$C_3$alkoxy, $-\underset{\underset{W}{\|}}{C}-R_8$, $-SO_2-NR_8R_7$,

$-SO_n-C_1-C_3$-alkyl or $-CO-R_9$,

$R_3$ is hydrogen, halogen, $C_1-C_3$alkyl, methoxy, nitro or trifluoromethyl,

$R_4$ is hydrogen, $C_1-C_4$alkyl, cyanoalkyl containing not more than 4 carbon atoms, $C_3-C_8$cycloalkyl, benzyl, $-CO-C_1-C_4$alkoxy, $-CO-NR_6R_7$, or $-CO-C_1-C_4$-alkyl which is unsubstituted or substituted by from 1 to 3 halogen atoms,

$R_5$ is hydrogen, nitro, fluorine, chlorine, bromine, methyl, trifluoromethyl, $-SO_n-C_1-C_3$alkyl, $-CO-C_1-C_4$alkoxy or $C_1-C_3$alkoxy,

$R_6$ is hydrogen, $C_1-C_6$alkyl, cyanoalkyl containing not more than 4 carbon atoms, methoxy or ethoxy,

$R_7$ is hydrogen, $C_1-C_6$alkyl or $C_3-C_6$alkenyl, or

$R_6$ and $R_7$, together with the nitrogen atom bonding them, form a 5- or 6-membered saturated heterocycle which may contain an oxygen or a sulfur atom as ring member,

$R_8$ is hydrogen, $C_1-C_6$alkyl, $C_1-C_4$haloalkyl, $C_3-C_6$-cycloalkyl, $C_4-C_7$cycloalkylalkyl or alkoxyalkyl containing not more than 4 carbon atoms,

$R_9$ is $C_1-C_6$alkoxy, $C_3-C_6$alkenyloxy, $C_3-C_6$alkynyloxy, $C_2-C_6$haloalkoxy, $C_1-C_4$cyanoalkoxy, $C_1-C_6$alkylthio, $C_3-C_6$alkenylthio, $C_3-C_6$alkynylthio, $C_5-C_6$cycloalkoxy, $C_4-C_7$cycloalkylalkoxy, $-NR_6R_7$ or alkoxyalkoxy containing not more than 6 carbon atoms,

W is oxygen or $=N-O-R_{10}$, wherein $R_{10}$ is hydrogen, $C_1-C_6$alkyl or $C_3-C_6$alkenyl, and

n is a value from 0 to 2,

and the salts of these compounds.

2. Compounds according to claim 1, characterised in that Z is oxygen.

3. Compounds according to claim 1, characterised in that Y is oxygen.

4. Compounds according to claim 1, characterised in that x is sulfur, $-NR_4-$ or $-CR_5=N-$.

5. Compounds according to claim 1, characterised in that E is the methine bridge -CH-.

6. Compounds according to claim 1, characterised in that $R_1$ is chlorine, dimethylamino, trifluoromethyl, fluoromethyl, methoxy, difluoromethoxy, methyl or ethyl.

7. Compounds according to claim 1, characterised in that $R_3$ is hydrogen.

8. Compounds according to claim 1, characterised in that the radical $R_2$ is vicinal to the sulfonyl group.

9. Compounds according to claim 8, characterised in that $R_2$ is hydrogen, fluorine, chlorine, methoxy, acetyl, $-SO_2-CH_3$, $-COOCH_2-CH=CH_2$, $-COOCH_2-C\equiv CH$, $-SO_2-N(CH_3)_2$ or $-CO-C_1-C_4$alkoxy.

10. Compounds according to claim 1, characterised in that Y and Z are oxygen, E is the methine bridge, $R_1$ is chlorine, dimetbylamino, trifluoromethyl, fluoromethyl, methoxy, difluoromethoxy, methyl or ethyl, $R_2$ is vicinal to the sulfonyl group and is hydrogen, fluorine, chlorine, methoxy, acetyl, $-SO_2-CH_3$, $-COOCH_2-CH=CH_2$, $-COOCH_2-C\equiv CH$, $-SO_2-N(CH_3)_2$ or $-CO-C_1-C_4$alkoxy, and $R_3$ is hydrogen.

11. Compounds according to claim 1, characterised in that X is a sulfur atom, Y and Z are oxygen, E is the methine bridge, $R_1$ is chlorine, dimethylamino, trifluoromethyl, fluoromethyl, methoxy, difluoromethoxy, methyl or ethyl, $R_2$ is vicinal to the sulfonyl group and is hydrogen, fluorine, chlorine, methoxy, acetyl, $-SO_2CH_3$, $-COOCH_2-CH=CH_2$, $-COOCH_2-C=CH$, $-SO_2-N(CH_3)_2$ or $-CO-CO_1-C_4$alkoxy and $R_3$ is hydrogen.

12. Compounds according to claim 1, characterised in that X is sulfur or $-CH=N-$, Y and Z are oxygen, E is the methine bridge, $R_1$ is chlorine, dimethylamino, fluoromethyl, methoxy, difluoromethoxy, methyl or ethyl, $R_2$ is vicinal to the sulfonyl group and is hydrogen, chlorine, $-COOCH_2-CH=CH_2$, $-COOCH_2-C\equiv CH$ or $-CO-C_1-C_4$alkoxy, and $R_3$ is hydrogen.

13. Compounds according to claim 1, characterised in that X is $-CR_5=N-$, Y and Z are oxygen, E is the methine bridge, $R_1$ is chlorine, dimethylamino, trifluoromethyl, fluoromethyl, methoxy, difluoromethoxy, methyl or ethyl, $R_2$ is vicinal to the sulfonyl group and is hydrogen, fluorine, chlorine, methoxy, acetyl, $-SO_2-CH_3$, $-COOCH_2-CH=CH_2$, $-COOCH_2-C\equiv CH$, $-SO_2-N(CH_3)_2$ or $-CO-C_1-C_4$alkoxy, and $R_3$ is hydrogen.

14. N-(2-Methoxycarbonyl-3-thienylsulfonyl)-N'-(4-difluoromethoxy-6-methylpyrimidin-2-yl)urea according to claim 1.

15. N-(4-Methoxycarbonyl-3-thienylsulfonyl)-N'-(4-difluoromethoxy-6-methylpyrimidin-2-yl)urea according to claim 1.

16. N-(2-Chloro-3-pyridinylsulfonyl)-N'-(4-difluoromethoxy-6-methylpyrimidin-2-yl)urea according to claim 1.

17. A process for the preparation of the compounds of the formula I according to claim 1, characterised in that either

a) a heterocyclosulfonamide of the formula II

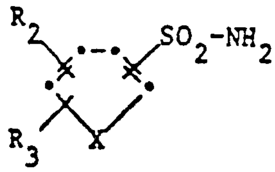

(II),

wherein $R_2$, $R_3$ and X are as defined for formula I, is reacted with an N-pyrimidinylcarbamate or N-triazinylcarbamate of the formula III

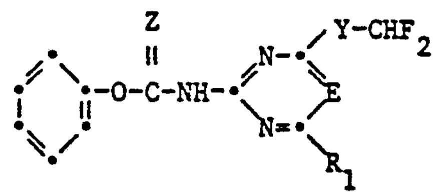

(III),

wherein E, $R_1$, Y and Z are as defined for formula I, in the presence of a base,
or
b) a heterocyclosulfonylisocyanate or heterocyclosulfonylisothiocyanate of the formula IV

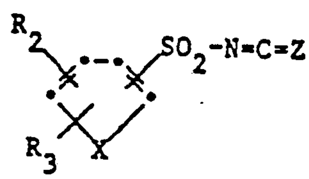

(IV),

wherein $R_2$, $R_3$, X and Z are as defined for formula I, is reacted with an aminopyrimidine or aminotriazine of the formula V

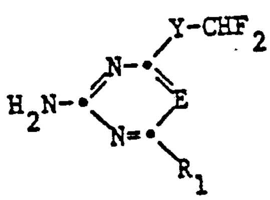

(V),

wherein E, $R_1$ and Y are as defined for formula I, optionally in the presence of a base,
or
c) a heterocyclosulfonamide of the formula II above is reacted with an isocyanate or isothiocyanate of the formula VI

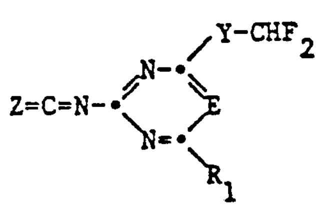

(VI),

wherein E, $R_1$, Y and Z are as defined for formula I, optionally in the presence of a base,
or
d) an N-heterocyclosulfonylcarbamate of the formula VII

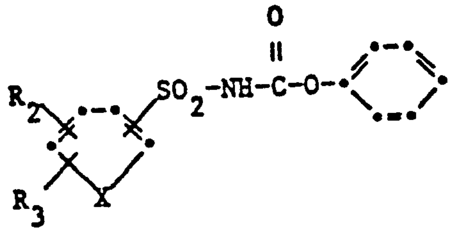

(VII),

wherein $R_2$, $R_3$ and X are as defined for formula I, is reacted with an aminopyrimidine or aminotriazine of the formula V above,

and, if desired, the sulfonylurea of the formula I is converted into a salt thereof by reaction with an amine, an alkali metal hydroxide or alkaline earth metal hydroxide, or with a quaternary ammonium base.

18. A herbicidal and plant growth-inhibiting composition, characterised in that it contains as active ingredient at least one N-heterocyclosulfonyl-N'-pyrimidinylurea or N-heterocyclosulfonyl-N'-triazinylurea of the formula I, claim 1, together with carriers and/or other adjuvants.

19. The use of the N-heterocyclosulfonyl-N'-pyrimidinylureas and N-heterocyclosulfonyl-N'-triazinylureas of the formula I, claim 1, or of compositions containing such compounds, for controlling undesired plant growth.

20. The use of the compounds of the formula I, claim 1, or of compositions containing such compounds, for regulating plant growth.

21. The use of the compounds of the formula I, or of compositions containing such compounds, according to claim 19, for selectively controlling weeds pre- or postemergence in crops of useful plants.

22. The use according to claim 21 for controlling weeds in crops of soybeans.

23. N-(4-Methoxycarbonyl-3-thienylsulfonyl)-N'-(4-difluoromethoxy-6-methoxypyrimidin-2-yl)urea according to claim 1.

**Claims** for the Contracting State: AT

1. A herbicidal and plant growth-inhibiting composition, characterised in that it contains as active ingredient, together with carriers and/or other adjuvants, at least one N-heterocyclosulfonyl-N'-pyrimidinylurea or N-heterocyclosulfonyl-N'-triazinylurea of the formula I

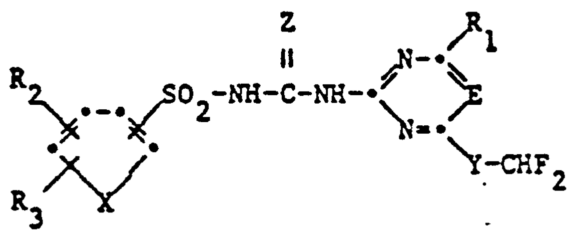

(I)

or a salt of these compounds, wherein

| | |
|---|---|
| X | is oxygen, sulfur, $-N(R_4)-$ or $-C(R_5)=N-$, |
| Y | is oxygen or sulfur, |
| Z | is oxygen or sulfur, |
| E | is nitrogen or -CH=, |
| $R_1$ | is hydrogen, $C_1$-$C_4$alkyl, $C_1$-$C_4$haloalkyl, $C_1$-$C_4$-haloalkoxy, $C_1$-$C_4$alkoxy, halogen, $-NR_6R_7$ or alkoxyalkyl containing not more than 4 carbon atoms, |
| $R_2$ | is hydrogen, $C_1$-$C_3$alkyl, $C_1$-$C_3$haloalkyl, halogen, nitro, $C_1$-$C_3$alkoxy, $-C(=W)-R_8$, $-SO_2-NR_8R_7$, $-SO_n-C_1-C_3$-alkyl or $-CO-R_9$, |
| $R_3$ | is hydrogen, halogen, $C_1$-$C_3$alkyl, methoxy, nitro or trifluoromethyl, |
| $R_4$ | is hydrogen, $C_1$-$C_4$alkyl, cyanoalkyl containing not more than 4 carbon atoms, $C_3$-$C_6$cycloalkyl, benzyl, $-CO-C_1-C_4$alkoxy, $-CO-NR_6R_7$, or $-CO-C_1-C_4$-alkyl which is unsubstituted or substituted by from 1 to 3 halogen atoms, |
| $R_5$ | is hydrogen, nitro, fluorine, chlorine, bromine, methyl, trifluoromethyl, $-SO_n-C_1-C_3$alkyl, $-CO-C_1-C_4$-alkoxy or $C_1$-$C_3$alkoxy, |

$R_6$ is hydrogen, $C_1$-$C_6$alkyl, cyanoalkyl containing not more than 4 carbon atoms, methoxy or ethoxy,
$R_7$ is hydrogen, $C_1$-$C_6$alkyl or $C_3$-$C_6$alkenyl, or
$R_6$ and $R_7$, together with the nitrogen atom bonding them, form a 5- or 6-membered saturated heterocycle which may contain an oxygen or a sulfur atom as ring member,
$R_8$ is hydrogen, $C_1$-$C_6$alkyl, $C_1$-$C_4$haloalkyl, $C_3$-$C_6$-cycloalkyl, $C_4$-$C_7$cycloalkylalkyl or alkoxyalkyl containing not more than 4 carbon atoms,
$R_9$ is $C_1$-$C_6$alkoxy, $C_3$-$C_6$alkenyloxy, $C_3$-$C_6$alkynyloxy, $C_2$-$C_6$haloalkoxy, $C_1$-$C_4$cyanoalkoxy, $C_1$-$C_6$alkylthio, $C_3$-$C_6$alkenylthio, $C_3$-$C_6$alkynylthio, $C_5$-$C_6$cycloalkoxy, $C_4$-$C_7$cycloalkylalkoxy, $-NR_6R_7$ or alkoxyalkoxy containing not more than 6 carbon atoms,
W is oxygen or $=N-O-R_{10}$, wherein $R_{10}$ is hydrogen, $C_1$-$C_6$alkyl or $C_3$-$C_6$alkenyl, and
n is a value from 0 to 2.

2. A composition according to claim 1, characterised in that Z is oxygen.

3. A composition according to claim 1, characterised in that Y is oxygen.

4. A composition according to claim 1, characterised in that X is sulfur, $-NR_4-$ or $-CR_5=N-$.

5. A composition according to claim 1, characterised in that E is the methine bridge $-CH=$.

6. A composition according to claim 1, characterised in that $R_1$ is chlorine, dimethylamino, trifluoromethyl, fluoromethyl, methoxy, difluoromethoxy, methyl or ethyl.

7. A composition according to claim 1, characterised in that $R_3$ is hydrogen.

8. A composition according to claim 1, characterised in that the radical $R_2$ is vicinal to the sulfonyl group.

9. A composition according to claim 8, characterised in that $R_2$ is hydrogen, fluorine, chlorine, methoxy, acetyl, $-SO_2-CH_3$, $-COOCH_2-CH=CH_2$, $-COOCH_2-C\equiv CH$, $-SO_2-N(CH_3)_2$ or $-CO-C_1$-$C_4$alkoxy.

10. A composition according to claim 1, characterised in that Y and Z are oxygen, E is the methine bridge, $R_1$ is chlorine, dimethylamino, trifluoromethyl, fluoromethyl, methoxy, difluoromethoxy, methyl or ethyl, $R_2$ is vicinal to the sulfonyl group and is hydrogen, fluorine, chlorine, methoxy, acetyl, $-SO_2-CH_3$, $-COOCH_2-CH=CH_2$, $-COOCH_2-C\equiv CH$, $-SO_2-N(CH_3)_2$ or $-CO-C_1$-$C_4$alkoxy, and $R_3$ is hydrogen.

11. A composition according to claim 1, characterised in that X is a sulfur atom, Y and Z are oxygen, E is the methine bridge, $R_1$ is chlorine, dimethylamino, trifluoromethyl, fluoromethyl, methoxy, difluoromethoxy, methyl or ethyl, $R_2$ is vicinal to the sulfonyl group and is hydrogen, fluorine, chlorine, methoxy, acetyl, $-SO_2-CH_3$, $-COOCH_2-CH=CH_2$, $-COOCH_2-C\equiv CH$, $-SO_2-N(CH_3)_2$ or $-CO-C_1$-$C_4$alkoxy, and $R_3$ is hydrogen.

12. A composition according to claim 1, characterised in that X is sulfur or $-CH=N-$, Y and Z are oxygen, E is the methine bridge, $R_1$ is chlorine, dimethylamino, fluoromethyl, methoxy, difluoromethoxy, methyl or ethyl, $R_2$ is vicinal to the sulfonyl group and is hydrogen, chlorine, $-COOCH_2-CH=CH_2$, $-COOCH_2-C\equiv CH$ or $-CO-C_1$-$C_4$alkoxy, and $R_3$ is hydrogen.

13. A composition according to claim 1, characterised in that X is $-CR_5=N-$, Y and Z are oxygen, E is the methine bridge, $R_1$ is chlorine, dimethylamino, trifluoromethyl, fluoromethyl, methoxy, difluoromethoxy, methyl or ethyl, $R_2$ is vicinal to the sulfonyl group and is hydrogen, fluorine, chlorine, methoxy, acetyl, $-SO_2-CH_3$, $-COOCH_2-CH=CH_2$, $-COOCH_2-C\equiv CH$, $-SO_2-N(CH_3)_2$ or $-CO-C_1$-$C_4$alkoxy, and $R_3$ is hydrogen.

14. A composition according to claim 1, characterised in that it contains N-(2-methoxycarbonyl-3-thienylsulfonyl)-N'-(4-difluoromethoxy-6-methylpyrimidin-2-yl)urea as active ingredient.

15. A composition according to claim 1, characterised in that it contains N-(4-methoxycarbonyl-3-thienylsulfonyl)-N'-(4-difluoromethoxy-6-methylpyrimidin-2-yl)urea as active ingredient.

16. A composition according to claim 1, characterised in that it contains N-(2-chloro-3-pyridinylsulfonyl)-N'-(4-difluoromethoxy-6-methylpyrimidin-2-yl)urea as active ingredient.

17. A process for the preparation of the compounds of the formula I according to claim 1, characterised in that either

a) a heterocyclosulfonamide of the formula II

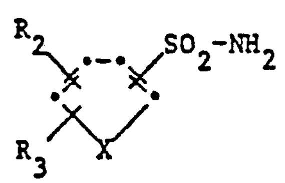

(II),

wherein $R_2$, $R_3$ and X are as defined for formula I, is reacted with an N-pyrimidinylcarbamate or N-triazinylcarbamate of the formula III

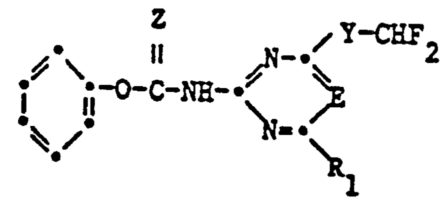

(III),

wherein E, $R_1$, Y and Z are as defined for formula I, in the presence of a base,

or

b) a heterocyclosulfonylisocyanate or heterocyclosulfonylisothiocyanate of the formula IV

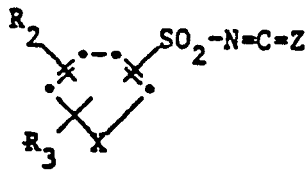

(IV),

wherein $R_2$, $R_3$, X and Z are as defined for formula I, is reacted with an aminopyrimidine or aminotriazine of the formula V

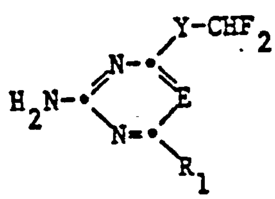

(V),

wherein E, $R_1$ and Y are as defined for formula I, optionally in the presence of a base, or

c) a heterocyclosulfonamide of the formula II above is reacted with an isocyanate or isothiocyanate of the formula VI

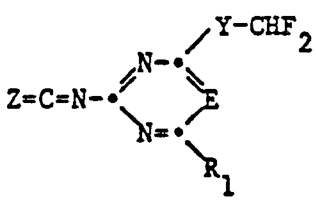

(VI),

wherein E, $R_1$, Y and Z are as defined for formula I, optionally in the presence of a base,

or

d) an N-heterocyclosulfonylcarbamate of the formula VII

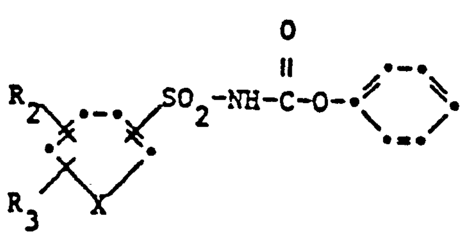

(VII),

wherein $R_2$, $R_3$ and X are as defined for formula I, is reacted with an aminopyrimidine or aminotriazine of the formula V above,

and, if desired, the sulfonylurea of the formula I is converted into a salt thereof by reaction with an amine, an alkali metal hydroxide or alkaline earth metal hydroxide, or with a quaternary ammonium base.

18. The use of the N-heterocyclosulfonyl-N'-pyrimidinylureas and N-heterocyclosulfonyl-N'-triazinylureas of the formula I, claim 1, or of compositions containing such compounds, for controlling undesired plant growth.

19. The use of the compounds of the formula I, claim 1, or of compositions containing such compounds, for regulating plant growth.

20. The use of the compounds of the formula I, or of compositions containing such compounds, according to claim 18, for selectively controlling weeds pre- or postemergence in crops of useful plants.

21. The use according to claim 20 for controlling weeds in crops of soybeans.

22. A composition according to claim 1, characterised in that it contains N-(4-methoxycarbonyl-3-thienylsulfonyl)-N'-(4-difluoromethoxy-6-methoxypyrimidin-2-yl)urea as active ingredient.

**Revendications** pour les Etats contractants: BE, CH, DE, FR, GB, IT, LI, NL, SE

1. N-hétérocyclosulfonyl-N'-pyrimidinyl- et -triazinyl-urées de formule générale I

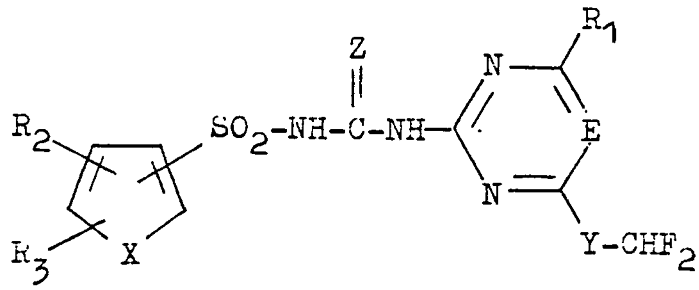

(I)

dans laquelle

| | |
|---|---|
| X | représente l'oxygène, le soufre, $-\underset{R_4}{\underset{\mid}{N}}-$ ou $-\underset{R_5}{\underset{\mid}{C}}=N-$ |
| Y | représente l'oxygène ou le soufre, |
| Z | représente l'oxygène ou le soufre, |
| E | représente l'azote ou -CH=, |
| $R_1$ | représente l'hydrogène, un groupe alkyle en $C_1$-$C_4$, halogénoalkyle en $C_1$-$C_4$, halogénoalcoxy en $C_1$-$C_4$, alcoxy en $C_1$-$C_4$, un halogène, un groupe $-NR_6R_7$ ou un groupe alcoxyalkyle contenant au maximum 4 atomes de carbone, |
| $R_2$ | représente l'hydrogène, un groupe alkyle en $C_1$-$C_3$, halogénoalkyle en $C_1$-$C_3$, un halogène, un groupe nitro, alcoxy en $C_1$-$C_3$, $-\underset{W}{\underset{\|}{C}}-R_8$, $-SO_2-NR_6R_7$, $-SO_n$-alkyle en $C_1$-$C_3$ ou $-CO-R_9$, |
| $R_3$ | représente l'hydrogène, un halogène, un groupe alkyle en $C_1$-$C_3$, méthoxy, nitro ou trifluorométhyle, |
| $R_4$ | représente l'hydrogène, un groupe alkyle en $C_1$-$C_4$, cyanalkyle à 4 atomes de carbone au maximum, cycloalkyle en $C_3$-$C_6$, benzyle, -CO-alcoxy en $C_1$-$C_4$, $-CO-NR_6R_7$ ou un groupe -CO-alkyle en $C_1$-$C_4$ éventuellement substitué par 1 à 3 atomes d'halogènes, |
| $R_5$ | représente l'hydrogène, un groupe nitro, le fluor, le chlore le brome un groupe méthyle, trifluorométhyle, $-SO_n$-alkyle en $C_1$-$C_3$, -CO-alcoxy en $C_1$-$C_4$ ou alcoxy en $C_1$-$C_3$, |
| $R_6$ | représente l'hydrogène, un groupe alkyle en $C_1$-$C_6$, cyanalkyle à 4 atomes de carbone au maximum, méthoxy ou éthoxy, |
| $R_7$ | représente l'hydrogène, un groupe alkyle en $C_1$-$C_6$ ou alcényle en $C_3$-$C_6$ ou bien |
| $R_6$ et $R_7$ | forment ensemble et avec l'atome d'azote auquel ils sont reliés un hétérocycle saturé à 5 ou 6 chaînons contenant éventuellement un atome d'oxygène ou de soufre en tant que chaînon cyclique, |
| $R_5$ | représente l'hydrogène, un groupe alkyle en $C_1$-$C_6$, halogénoalkyle en $C_1$-$C_4$, cycloalkyle en $C_3$-$C_6$, cycloalkylalkyle en $C_4$-$C_7$ ou alcoxyalkyle à 4 atomes de carbone au maximum, |
| $R_9$ | représente un groupe alcoxy en $C_1$-$C_6$, alcényloxy en $C_3$-$C_6$, alcynyloxy en $C_3$-$C_6$, halogénoalcoxy en $C_2$-$C_6$, cyanoalcoxy en $C_1$-$C_4$, alkylthio en $C_1$-$C_6$, alcénylthio en $C_3$-$C_6$, alcynylthio en $C_3$-$C_6$, cycloalcoxy en $C_5$-$C_6$, cycloalkylalcoxy en $C_4$-$C_7$, $-NR_6R_7$ ou alcoxyalcoxy à 6 atomes de carbone au maximum, |
| W | représente l'oxygène ou $=N-O-R_{10}$ dans lequel $R_{10}$ représente l'hydrogène, un groupe alkyle en $C_1$-$C_6$ ou alcényle en $C_3$-$C_6$, et |
| n | est un nombre de 0 à 2, ainsi que les sels de ces composes. |

2. Composés selon la revendication 1, caractérisé em ce que Z représente l'oxygène.

3. Composés selon la revendication 1, caractérisés en ce que Y représente l'oxygène.

4. Composés selon la revendication 1, caractérisés en ce que X représente le soufre, $-NR_4-$ ou $-CR_5=N-$.

5. Composés selon la revendication 1, caractérisés en ce que E représente le pont méthine $-CH=$.

6. Composés selon la revendication 1, caractérisés en ce que $R_1$ représente le chlore, un groupe diméthylamino, trifluorométhyle, fluorométhyle, méthoxy, difluorométhoxy, méthyle ou éthyle.

7. Composés selon la revéndication 1, caractérisé en ce que $R_3$ représente l'hydorgène.

8. Composés selon la revendication 1, caractérisés en ce que le substituant $R_2$ est en position voisine du groupe sulfonyle.

9. Composés selon la revendication 8, caractérisés en ce que $R_2$ représente l'hydrogène, le fluor, le chlore, un groupe méthoxy, acétyle, $-SO_2-CH_3$, $-COOCH_2-CH=CH_2$, $-COOCH_2-C\equiv CH$, $-SO_2-N(CH_3)_2$ ou -CO-alcoxy en $C_1$-$C_4$.

10. Composés selon la revendication 1, caractérisés en ce que Y et Z représentent l'oxygène, E le pont méthine, $R_1$ le chlore, un groupe diméthylamino, trifluorométhyle, fluorométhyle, méthoxy, difluorométhoxy, méthyle ou éthyle, $R_2$ est en position voisine du groupe sulfonyle et représente l'hydrogène, le fluor, le chlore, un groupe méthoxy, acétyle, $SO_2-CH_3$, $-COOCH_2-CH=CH_2$, $-COOCH_2-C\equiv CH$, $-SO_2-N(CH_3)_2$ ou -CO-alcoxy en $C_1$-$C_4$, et $R_3$ représente l'hydrogène.

11. Composés selon la revendication 1, caractérisés en ce que X représente un atome de soufre, Y et Z représentent l'oxygène, E le pont méthine, $R_1$ le chlore, un groupe diméthylamino, trifluorométhyle, fluorométhyle, méthoxy, difluorométhoxy, méthyle ou éthyle, $R_2$ est en position voisine du groupe sulfonyle et représente l'hydrogène, le fluor, le chlore, un groupe méthoxy, acétyle, $-SO_2-CH_3$, $-COOCH_2-CH=CH_2$, $-COOCH_2-C\equiv CH$, $-SO_2-N(CH_3)_2$ ou -CO-alcoxy en $C_1$-$C_4$ et $R_3$ représente l'hydrogène.

12. Composés selon la revendication 1, caractérisés en ce que X représente le soufre ou $-CH=N-$, Y et Z représentent l'oxygène, E le pont méthine, $R_1$ représente le chlore, un groupe diméthylamino, fluorométhyle, méthoxy, difluorométhoxy, méthyle ou éthyle, $R_2$ est en position voisine du groupe sulf onyle et représente l'hydrogène, le chlore, un groupe $-COOCH_2-CH=CH_2$, $-COOCH_2-C\equiv CH$ ou -CO-alcoxy en $C_1$-$C_4$ et $R_3$ représente l'hydrogène.

13. Composés selon la revendication 1, caractérisés en ce que X représente $-CR_5=N-$, Y et Z représentent l'oxygène, E le pont méthine, $R_1$ le chlore, un groupe diméthylamino, trifluorométhyle, fluorométhyle, méthoxy, difluorométhoxy, méthyle ou éthyle, $R_2$ est en position voisine du groupe sulfonyle et représente l'hydrogène, le fluor, le chlore, un groupe méthoxy, acétyle, $-SO_2-CH_3$, $-COOCH_2-CH=CH_2$, $-COOCH_2-C\equiv CH$, $-SO_2-N(CH_3)_2$ ou -CO-alcoxy en $C_1$-$C_4$ et $R_3$ représente l'hydrogène

14. La N-(2-méthoxycarbonyl-3-thiénylsulfonyl)-N'-(4-difluorométhoxy-6-méthyl-pyrimidine-2-yl)-urée selon la revendication 1.

15. La N-(4-méthoxycarbonyl-3-thiénylsulfonyl)-N'-(4-difluorométhoxy-6-méthyl-pyrimidine-2-yl)-urée selon la revendication 1.

16. La N-(2-chloro-3-pyridinylsulfonyl)-N'-(4-difluorométhoxy-6-méthylpyrimidine-2-yl)-urée selon la revendication 1.

17. Procédé de préparation des composés de formule I selon la revendication 1, caractérisé en ce que:

a) on fait réagir un hétérocyclosulfonamide de formule II

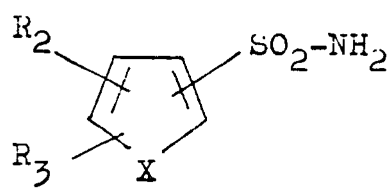

(II),

dans laquelle $R_2$, $R_3$ et X ont les significations indiquées en référence à la formule I, en précence d'une base, avec un N-pyrimidinyl- ou -triazinyl-carbamate de formule III

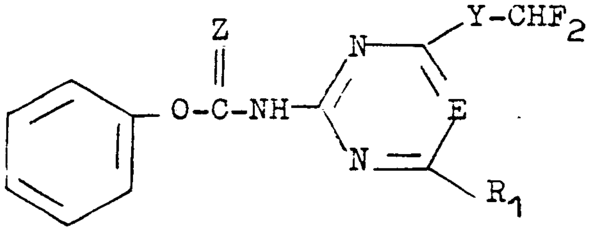

(III),

dans laquelle E, $R_1$, Y et Z ont les significations indiquées en référence à la formule I, ou bien

b) on fait réagir un hétérocyclosulfonylisocyanate ou -isothiocyanate de formule IV

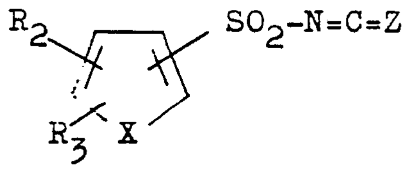

(IV),

indiquées en référence à la formule I, éventuellement en présence d'une base, avec une aminopyrimidine ou -triazine de formule V

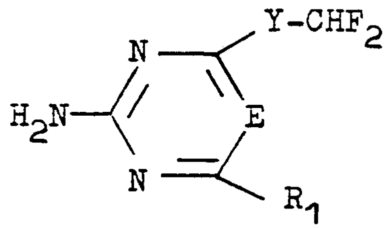

(V),

dans laquelle E, $R_1$ et Y ont les significations indiquées en référence à la formule I, ou bien

c) on fait réagir un hétérocyclosulfonamide de formule II ci-dessus éventuellement en présence d'une base avec un isocyanate ou isothiocyanate de formule VI

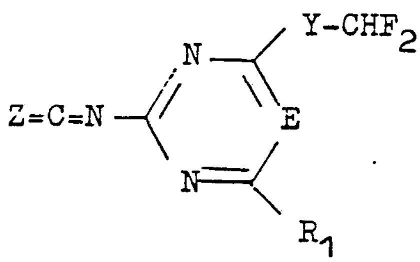

(VI)

dans laquelle E, $R_1$, Y et Z ont les significations indiquées en référence à la formule I, ou bien

d) on fait réagir un N-hétérocyclosulfonylcarbamate de formule VII

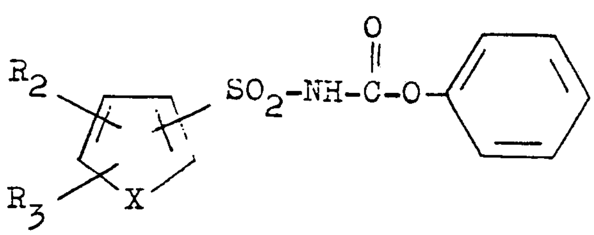

(VII)

dans laquelle $R_2$, $R_3$ et X ont les significations indiqué es en référence à la formule I, avec une aminopyrimidine ou -triazine de formule V ci-dessus et le cas échéant on convertit en les sels en faisant réagir une sulfonylurée de formule I avec une amine, un hydroxyde de métal alcalin ou alcalino-terreux ou une base d'ammonium quaternaire.

18. Un produit herbicide et inhibiteur de la croissance des végétaux, caractérisé en ce qu'il contient en tant que substance active, avec des véhicules et/ou d'autres additifs, au moins une N-hétérocyclosulfonyl-N'-pyrimidinyl- ou -triazinyl-urée de formule I, revendication 1.

19. L'utilisation des N-hétérocyclosulfonyl-N'-pyrimidinyl- et -triazinyl-urées de formule I, revendication 1, ou de produits en contenant dans la lutte contre les croissances de végétaux indésirables.

20. L'utilisation des composés de formule I, revendication 1, ou de produits en contenant pour la régulation de la croissance des végétaux.

21. L'utilisation des composés de formule I ou de produits en contenant, selon la revendication 19, pour la lutte sélective en pré-levée ou en post-levée contre les mauvaises herbes dans les cultures de végétaux utiles.

22. L'utilisation selon la revendication 21, pour la lutte contre les mauvaises herbes dans les cultures de soja.

23. La N-(4-méthoxycarbonyl-3-thiénylsulfonyl)-N'-(4-difluorométhoxy-6-méthoxy-pyrimidine-2-yl)-urée selon la revendication 1.

**Revendications** pour l'Etat contractant: AT

1. Un produit herbicide et inhibiteur de la croissance des végétaux, caractérisé en ce qu'il contient en tant que substance active, avec des véhicules et/ou d'autres additifs, une N-hétérocyclosulfonyl-N'pyrimidinyl- ou -triazinyl-urée de formule

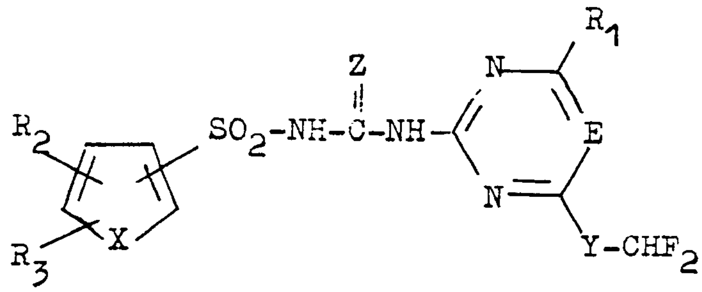
(I)

ou un sel d'un tel composé,
dans lequel

X représente l'oxygène, le soufre, $-\underset{R_4}{\underset{|}{N}}-$ ou $-\underset{R_5}{\underset{|}{C}}=N-$

Y représente l'oxygène ou le soufre,
Z représente l'oxygène ou le soufre,
E représente l'azote ou -CH=,
$R_1$ représente l'hydrogène, un groupe alkyle en $C_1$-$C_4$, halogénoalkyle en $C_1$-$C_4$, halogénoalcoxy en $C_1$-$C_4$, alcoxy en $C_1$-$C_4$, un halogène, un groupe $-NR_6R_7$ ou un groupe alcoxyalkyle à 4 atomes de carbone au maximum.
$R_2$ représente l'hydrogène, un groupe alkyle en $C_1$-$C_3$, halogénoalkyle en $C_1$-$C_3$, un halogène, un groupe nitro, alcoxy en $C_1$-$C_3$, $-\underset{W}{\underset{\|}{C}}-R_8$, $-SO_2-NR_6R_7$, $-SO_n$-alkyle en $C_1$-$C_3$ ou $-CO-R_9$,
$R_3$ représente l'hydrogène, un halogène, un groupe alkyle en $C_1$-$C_3$, méthoxy, nitro ou trifluorométhyle,
$R_4$ représente l'hydrogène, un groupe alkyle en $C_1$-$C_4$, cyanalkyle à 4 atomes de carbone au maximum, cycloalkyle en $C_3$-$C_6$ benzyle, -CO-alcoxy en $C_1$-$C_4$, $-CO-NR_6R_7$ ou un groupe -CO-alkyle en $C_1$-$C_4$ éventuellement substitué par 1 à 3 atomes d'halogènes,
$R_5$ représente l'hydrogène, un groupe nitro, le fluor, le chlore, le brome, un groupe méthyle, trifluorométhyle, $-SO_n$-alkyle en $C_1$-$C_3$, -CO-alcoxy en $C_1$-$C_4$ ou alcoxy en $C_1$-$C_3$,
$R_6$ représente l'hydrogène, un groupe alkyle en $C_1$-$C_6$, cyanalkyle à 4 atomes de carbone au maximum, méthoxy ou éthoxy,
$R_7$ représente l'hydrogène, un groupe alkyle en $C_1$-$C_6$ ou alcényle en $C_3$-$C_6$ ou bien
$R_6$ et $R_7$ forment ensemble et avec l'atome d'azote auquel ila sont reliés, un hétérocycle saturé à 5 ou 6 chaînons contenant le cas échéant un atome d'oxygène ou de soufre en tant que chaînon cyclique,
$R_8$ représente l'hydrogène, un groupe alkyle en $C_1$-$C_6$, halogénoalkyle en $C_1$-$C_4$, cycloalkyle en $C_3$-$C_6$, cycloalkylalkyle en $C_4$-$C_7$ ou alcoxyalkyle contenant 4 atomes de carbone au maximum,
$R_9$ représente un groupe alcoxy en $C_1$-$C_6$, alcényloxy en $C_3$-$C_6$, alcynyloxy en $C_3$-$C_6$, halogénoalcoxy en $C_2$-$C_6$, cyanalcoxy en $C_1$-$C_4$, alkylthio en $C_1$-$C_6$, alcénylthio en $C_3$-$C_6$, alcynylthio en $C_3$-$C_6$, cycloalcoxy en $C_5$-$C_6$, cycloalkylalcoxy en $C_4$-$C_7$, $-NR_6R_7$ ou alcoxyalcoxy à 6 atomes de carbone au maximum,
W représente l'oxygène ou $=N-O-R_{10}$ dans lequel $R_{10}$ représente l'hydrogène, un groupe alkyle en $C_1$-$C_6$ ou alcenyle en $C_3$-$C_6$, et
n est un nombre allant de 0 à 2.

2. Produit selon la revendication 1, caractérisé en ce que Z représente l'oxygène.
3. Produit selon la revendication 1, caractérisé en ce que Y représente l'oxygène.
4. Produit selon la revendication 1, caractérisé en ce que X représente le soufre, $-NR_4-$ ou $-CR_5=N-$.
5. Produit selon la revendication 1, caractérisé en ce que E représente le pont méthine -CH=.
6. Produit selon la revendication 1, caractérisé en ce que $R_1$ représente le chlore, un groupe diméthylamino, trifluorométhyle, fluorométhyle, méthoxy, difluorométhoxy, méthyle ou éthyle.
7. Produit selon la revendication 1, caractérisé en ce que $R_3$ représente l'hydrogène.
8. Produit selon la revendication 1, caractérisé en ce que le substituant $R_2$ est en position voisine du groupe sulfonyle.
9. Produit selon la revendication 8, caractérisé en ce que $R_2$ représente l'hydrogène, le fluor, le chlore, un groupe méthoxy, acétyle, $-SO_2-CH_3$, $-COOCH_2-CH=CH_2$, $-COOCH_2-C\equiv CH$, $-SO_2-N(CH_3)_2$ ou -CO-alcoxy en $C_1$-$C_4$.

10. Produit selon la revendication 1, caractérisé en ce que Y et Z représentent l'oxygène, E le pont méthine, $R_1$ le chlore, un groupe diméthylamino, trifluorométhyle, fluorométhyle, méthoxy, difluorométhoxy, méthyle ou éthyle, $R_2$ est em position voisine du groupe sulfonyle et représente l'hydrogène, le fluor, le chlore, un groupe méthoxy, acétyle, $-SO_2-CH_3$, $-COOCH_2-CH=CH_2$, $-COOCH_2-C\equiv CH$, $-SO_2-N(CH_3)_2$ ou -CO-alcoxy en $C_1$-$C_4$ et $R_3$ représente l'hydrogène.

11. Produit selon la revendication 1, caractérisé en ce que X représente un atome de soufre, Y et Z l'oxygène, E le pont méthine, $R_1$ le chlore, un groupe diméthylamino, trifluorométhyle, fluorométhyle, méthoxy, difluorométhoxy, méthyle ou éthyle, $R_2$ est en position voisine du groupe sulfonyle et représente l'hydrogène, le fluor, le chlore, un groupe méthoxy, acétyle, $-SO_2-CH_3$, $-COOCH_2-CH=CH_2$, $-COOCH_2-C\equiv CH$, $-SO_2-N(CH_3)_2$ ou -CO-alcoxy en $C_1$-$C_4$ et $R_3$ représente l'hydrogène.

12. Produit selon la revendication 1, caractérisé en ce que X représente le soufre ou $-CH=N-$, Y et Z représentent l'oxygène, E le pont méthine, $R_1$ le chlore, un groupe diméthylamino, fluorométhyle, méthoxy, difluorométhoxy, méthyle ou éthyle, $R_2$ est en position voisine du groupe sulfonyle et représente l'hydrogène, le chlore, un groupe $-COOCH_2-CH=CH_2$, $-COOCH_2-C\equiv CH$ ou -CO-alkoxy en $C_1$-$C_4$ et $R_3$ représente l'hydrogène.

13. Produit selon la revendication 1, caractérisé en ce que X représente $-CR_5=N-$, Y et Z représentent l'oxygène, E le pont méthine, $R_1$ le chlore, un groupe diméthylamino, trifluorométhyle, fluorométhyle, méthoxy, difluorométhoxy, méthyle ou éthyle, $R_2$ est en position voisine du groupe sulfonyle et représente l'hydrogène, le fluor, le chlore, un groupe méthoxy en $C_1$-$C_3$ acétyle, $-SO_2-CH_3$, $-COOCH_2-CH=CH_2$, $-COOCH_2-C\equiv CH$, $-SO_2-N(CH_3)_2$ ou -CO-alcoxy en $C_1$-$C_4$ et $R_3$ représente l'hydrogène.

14. Produit selon la revendication 1, caractérisé em ce qu'il contient en tant que substance active la N-(2-méthoxycarbonyl-3-thiénylsulfonyl)-N'-(4-difluorométhoxy-6-méthoxypyrimidine-2-yl)-urée.

15. Produit selon la revendication 1, caractérisé en ce qu'il contient en tant que substance active la N-(4-méthoxycarbonyl-3-thiénylsulfonyl)-N'-(4-difluorméthoxy-6-méthylpyrimidine-2-yl)-urée.

16. Produit selon la revendication 1, caractérisé en ce qu'il contient en tant que substance active la N-(2-chloro-3-pyridinylsulfomyl)-N'-(4-difluorométhoxy-6-méthoxypyrimidine-2-yl)-urée.

17. Procédé de préparation des composés de formule I selon la revendication 1, caractérisé en ce que:

a) on fait réagir un hétérocyclosulfonamide de formule II

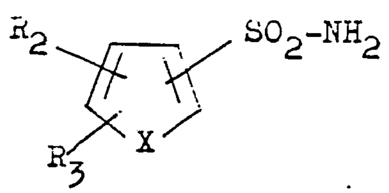

(II),

dans laquelle $R_2$, $R_3$ et X ont les significatioms indiquées en référence à la formule I, en présence d'une base, avec un N-pyrimidinyl- ou triazinyl-carbamate de formule III

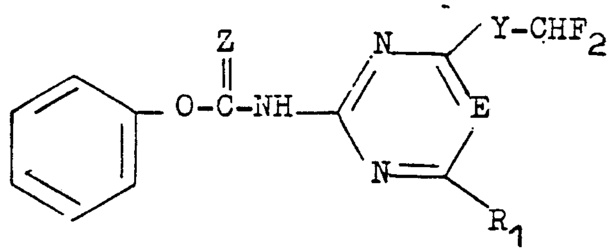

(III),

dans laquelle E, $R_1$, Y et Z ont les significations indiquées en référence à la formule I, ou bien

b) on fait réagir un hétérocyclosulfonylisocyanate ou -isothiocyanate de formule IV

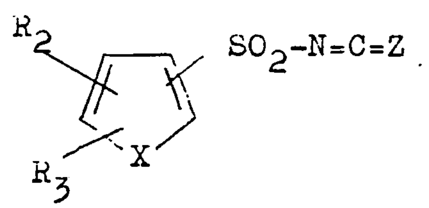

(IV)

dans laquelle $R_2$, $R_3$, X et Z ont les significations indiquées en référence à la formule I, éventuellement en présence d'une base, avec une aminopyrimidine ou -triazine de formule V

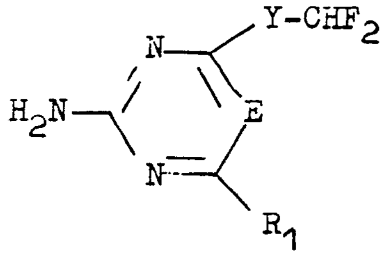

(V),

dans laquelle E, $R_1$ et Y ont les significations indiquées en référence à la formule I, ou bien

c) on fait réagir un hétérocyclosulfonamide de formule II ci-dessus éventuellement en présence d'une base avec un isocyanate ou isothiocyanate de formule VI

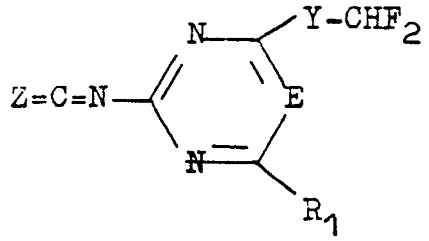

(VI),

dans laquelle E, $R_1$ et Y ont les significations indiquées en référence à la formule I, ou bien

d) on fait réagir un N-hétérocyclosulfonylcarbamate de formule VII

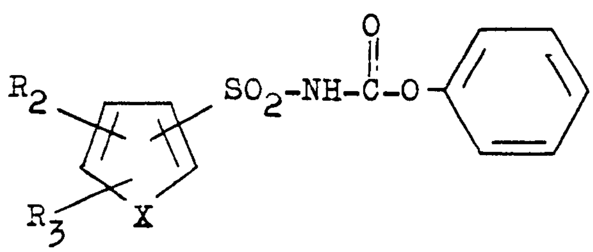

(VII)

dans laquelle $R_2$, $R_3$ et X ont les significations indiquées en référence à la formule I, avec une aminopyrimidine ou -triazine de formule V ci-dessus et le cas échéant on convertit en les sels en faisant réagir une sulfonylurée de formule I avec une amine, un hydroxyde de métal alcalin ou alcalino-terreux ou une vase d'ammonium quaternaire.

18. L'utilisation des N-hétérocyclosulfonyl-N'-pyrimidinyl- ou -triazinyl-urées de formule I, revendication 1, ou de produits en contenant, dans la lutte contre les croissances de végétaux indésirables.

19. L'utilisation des composés de formule I, revendication 1, ou de produits en contenant, pour la régulation de la croissance des végétaux.

20. L'utilisation des composés de formule I ou de produits en contenant selon la revendication 18, pour la lutte sélective en pré-levée ou en post-levée contre les mauvaises herbes dans les cultures de végétaux utiles.

21. L'utilisation selon la revendication 20, dans la lutte contre les mauvaises herbes dans les cultures de soja.

22. Produit selon la revendication 1, caractérisé en ce qu'il contient en tant que substance active la N-(4-méthoxycarbonyl-3-thiénylsulfonyl)-N'-(4-difluorométhoxy-6-méthoxypyrimidine-2-yl)-urée.